(19) Europäisches Patentamt
European Patent Office
Office européen des brevets

(11) **EP 2 305 033 A1**

(12) **EUROPÄISCHE PATENTANMELDUNG**

(43) Veröffentlichungstag:
06.04.2011 Patentblatt 2011/14

(51) Int Cl.:
*A01N 47/24* *(2006.01)*    *C07D 231/22* *(2006.01)*

(21) Anmeldenummer: **10175103.0**

(22) Anmeldetag: **19.06.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL HR MK RS**

(30) Priorität: **20.06.2005  DE 102005028493**

(62) Dokumentnummer(n) der früheren Anmeldung(en) nach Art. 76 EPÜ:
**06762088.0 / 1 937 071**

(71) Anmelder: **BASF SE**
**67056 Ludwigshafen (DE)**

(72) Erfinder:
• **Ziegler, Hans**
**67112 Mutterstadt (DE)**
• **Mayer, Winfried**
**55270 Bubenheim (DE)**
• **Kröhl, Thomas**
**69198 Schriesheim (DE)**
• **Schneider, Karl-Heinrich**
**67271 Kleinkarlbach (DE)**

• **Cox, Gerhard**
**67098 Bad Dürkheim (DE)**
• **Erk, Peter**
**67227 Frankenthal (DE)**
• **Vogelbacher, Uwe Josef**
**67071 Ludwigshafen (DE)**
• **Götz, Roland**
**68809 Neulußheim (DE)**
• **Wuckelt, Jörg**
**01987 Schwarzheide (DE)**
• **Rauls, Matthias**
**67061 Ludwigshafen (DE)**
• **Noack, Rainer**
**04932 Grossthiemig (DE)**

(74) Vertreter: **Reitstötter - Kinzebach**
**Patentanwälte**
**Ludwigsplatz 4**
**67059 Ludwigshafen (DE)**

Bemerkungen:
Diese Anmeldung ist am 02-09-2010 als Teilanmeldung zu der unter INID-Code 62 erwähnten Anmeldung eingereicht worden.

(54) **Kristalline Modifikationen des Pyraclostrobins**

(57) Die vorliegende Erfindung betrifft neue kristalline Modifikationen des Pyraclostrobins, Verfahren zu ihrer Herstellung und die Verwendung der neuen Modifikationen zur Herstellung von Pflanzenschutzmitteln.

Abbildung 2: Pulverdiffraktogramm von Pyraclostrobin Modifikation II

EP 2 305 033 A1

**Beschreibung**

Die vorliegende Erfindung betrifft neue kristalline Modifikationen des Pyraclostrobins, Verfahren zu ihrer Herstellung und die Verwendung der neuen Modifikationen zur Herstellung von Pflanzenschutzmitteln.

Pyraclostrobin (Methyl N-[[[1-(4-chlorphenyl)pyrazol-3-yl]oxy]-o-tolyl]-N-methoxy-carbamat) ist ein Wirkstoff zur Bekämpfung von phytopathogenen Pilzen (siehe z.B. WO 96/01256 und Herms, S., Seehaus, K., Koehle, H., and Conrath, U. (2002) Pyraclostrobin - "More than just a Fungicide" Phytomedizin 32: 17). Bei dem im Markt verfügbaren Pyraclostrobin handelt es sich um eine amorphe, niedrig schmelzende Substanz. Aufgrund dieser Eigenschaft eignet sich das kommerziell erhältliche Pyraclostrobin nicht zur Herstellung wässriger Suspensionskonzentrate (SC) auf konventionelle Weise, da beim Vermahlen die Mahlvorrichtungen verkleben. Aus diesem Grunde war auch die kommerzielle Herstellung von biologisch und wirtschaftlich interessanten Mischprodukten des Pyraclostrobins mit anderen Pflanzenschutzwirkstoffen in Form von Suspensionskonzentraten auf konventionellem Wege bislang nicht möglich.

Aus diesem Grund wird Pyraclostrobin häufig in Form von lösungsmittelhaltigen Emulsionskonzentraten oder Suspoemulsionskonzentraten oder in Form von wasserdispergierbaren Granulaten formuliert. Emulsionskonzentrate und Suspoemulsionskonzentrate enthalten jedoch größere Mengen an organischen, mit Wasser nicht mischbaren Lösungsmitteln, z.B. aromatische Kohlenwasserstoffe, so dass diese Formulierungen sowohl aus Gründen des Umweltschutzes und der Arbeitshygiene problematisch sind. Zudem kann bei Suspoemulsionskonzentraten des Pyraclostrobins unter bestimmten Bedingungen während der Lagerung eine Abscheidung von Wirkstoffpartikeln auftreten.

Die WO 03/082013 schlägt vor, Wirkstoffpartikel durch Aufziehen einer Pyraclostrobin-Schmelze auf ein Trägermaterial herzustellen. Aus den dabei resultierenden Adsorbaten können nach üblichen Verfahren Suspensionskonzentrate hergestellt werden, in die auch Mischungspartner eingebracht werden können. Allerdings kann bei diesen Suspensionskonzentraten nach einiger Zeit, insbesondere bei Lagerung bei höherer Temperatur, eine irreversible Partikelvergrößerung der dispergierten Wirkstoffpartikel auftreten. Hierdurch wird die Qualität des Produktes stark beeinträchtigt. Zudem ist das Verfahren vergleichsweise aufwändig, da es zusätzliche Materialien und Prozessschritte erfordert.

Der vorliegenden Erfindung liegt daher die Aufgabe zugrunde, Pyraclostrobin in einer Form bereitzustellen, welche die Herstellung von Suspensionskonzentraten mit verbesserter Stabilität erlaubt.

Diese Aufgabe wird durch die im Folgenden näher beschriebenen kristallinen Modifikationen II und IV des Pyraclostrobins gelöst.

Die Erfindung betrifft somit zum einen eine kristalline Modifikation IV des Pyraclostrobins, die in einem Röntgenpulverdiffraktogramm bei 25°C wenigstens drei, insbesondere wenigstens 4 und besonders bevorzugt alle der folgenden Reflexe zeigt:

$$d = 6,02 \pm 0,01 \text{ Å}$$
$$d = 4,78 \pm 0,01 \text{ Å}$$
$$d = 4,01 \pm 0,01 \text{ Å}$$
$$d = 3,55 \pm 0,01 \text{ Å}$$
$$d = 3,01 \pm 0,01 \text{ Å}.$$

Kristallines Pyraclostrobin der Modifikation IV weist typischerweise einen Schmelzpunkt im Bereich von 62 bis 72°C, insbesondere im Bereich von 64 bis 68°C und speziell im Bereich von 65 bis 67°C auf. Die Schmelzwärme, d.h. die Energiemenge, die zum Schmelzen der kristallinen Modifikation IV benötigt wird, beträgt etwa 72 bis 78 J/g und insbesondere etwa 74 ± 1 J/g. Die hier angegebenen Schmelzpunkte und Schmelzwärmen beziehen sich auf mittels Differentialkalorimetrie (Differential Scanning Calorimetry: DSC, Tiegelmaterial Aluminium, Aufheizrate 5 K/min) ermittelte Werte.

Untersuchungen an Einkristallen der Modifikation IV zeigen, dass die zugrunde liegende Kristallstruktur monoklin ist und die Raumgruppe P2(1)/c aufweist. Die charakteristischen Daten der Kristallstruktur von Modifikation IV sind in der Tabelle 1 angegeben:

Tabelle 1:Kristallographische Daten der Modifikation IV

| Parameter | Modifikation IV |
| --- | --- |
| Klasse | Monoklin |

(fortgesetzt)

| Parameter | Modifikation IV |
|---|---|
| Raumgruppe | P2(1)/c |
| a | 998,5(3) pm |
| b | 4780,4(10) pm |
| c | 788,6(2) pm |
| $\alpha$ | 90° |
| $\beta$ | 105,357(6) ° |
| $\gamma$ | 90° |
| Volumen | 3,6301 (16) $nm^3$ |
| Z | 8 |
| Dichte (berechnet) | 1,419 $g/cm^3$ |
| R1, wR2 | 0,0651, 0,1574 |
| a,b,c = Kantenlänge der Elementarzelle $\alpha$ ,$\beta$,$\gamma$ = Winkel der Elementarzelle Z = Anzahl der Moleküle in der Elementarzelle | |

Die Herstellung der kristallinen Modifikation IV des Pyraclostrobins gelingt durch ein Verfahren (im Folgenden auch Verfahren IVa), das die folgenden Schritte umfasst:

i) Lösen einer von Modifikation IV verschiedenen Form des Pyraclostrobins in einem organischen Lösungsmittel oder Lösungsmittelgemisch, wobei das organische Lösungsmittel oder Lösungsmittelgemisch wenigstens 70 Vol.-% wenigstens eines mit Wasser vollständig mischbaren organischen Lösungsmittels L1 und gegebenenfalls bis zu 30 Vol.-% Wasser enthält; und

ii) Bewirken einer Kristallisation von Pyraclostrobin über einen Zeitraum von wenigstens 10 h, insbesondere wenigstens 15 h und speziell wenigstens 20 h und/oder in Gegenwart von Impfkristallen der Modifikation IV.

Als Formen des Pyraclostrobins, die von der Modifikation IV verschieden sind, kommen beispielsweise eine feste oder flüssige Pyraclosstrobin-Schmelze, amorphes Pyraclostrobin oder ein Pyraclostrobin der Modifikationen I, II oder III, oder Mischungen davon in Betracht. In einer bevorzugten Ausführungsform löst man eine Schmelze des Pyraclostrobins in einem der vorgenannten organischen Lösungsmittel bzw. Lösungsmittelgemische. Das eingesetzte Pyraclostrobin weist vorzugsweise eine Reinheit von wenigstens 90 %, insbesondere wenigstens 95 % und speziell wenigstens 98 % auf.

Bei dem Lösungsmittel L1 kann es sich um ein reines Lösungsmittel L1 oder um eine Mischung verschiedener Lösungsmittel L1 handeln. Erfindungsgemäß ist das Lösungsmittel L1 mit Wasser vollständig mischbar. Hierunter versteht man, dass das Lösungsmittel mit Wasser bei 25°C (und 1023 mbar) vollständig mischbar ist, d.h. bei der genannten Temperatur mit Wasser keine Mischungslücke aufweist. Bevorzugt sind solche Lösungsmittel L1, die über einen größeren Temperaturbereich, insbesondere im gesamten Temperaturbereich, der für die Kristallisation relevant ist, d.h. im Bereich von 0 bis 80°C, zumindest aber im Temperaturbereich von 10 bis 60°C bei 1023 mbar mit Wasser vollständig mischbar sind, d.h. in diesem Temperaturbereichen keine Mischungslücke mit Wasser aufweisen. Geeignete Lösungsmittel sind dem Fachmann geläufig und können der Fachliteratur und entsprechenden Nachschlagewerken wie Handbook of Chemistry and Physics, CRC Pres, Ullmanns Encyclopedia of Industrial Chemistry, 5th ed. on CD ROM, Wiley-VCH, 1997 (Kapitel Solvents) sowie Industrial Solvents Handbook, 2nd ed. Marcel Dekker 2003, entnommen werden. Bevorzugt werden weiterhin Lösungsmittel L1, deren Siedepunkt bei Normaldruck im Bereich von 50 bis 100°C liegt.

Bevorzugte Lösungsmittel L1 sind $C_1$-$C_4$-Alkanole wie Methanol, Ethanol, n-Propanol, Isopropanol, n-Butanol, Isobutanol, 2-Butanol und tert.-Butanol, sowie Aceton und Butanon und deren Mischungen. Besonders bevorzugte Lösungsmittel L1 sind Methanol, Ethanol, n-Propanol und Isopropanol und deren Mischungen und speziell Methanol und Ethanol und deren Mischungen, weiterhin Aceton und Butanon.

Neben dem Lösungsmittel L1 kann das zum Lösen des Pyraclostrobins eingesetzte Lösungsmittel bzw. Lösungsmittelgemisch weitere, von L1 verschieden Lösungsmittel enthalten. Typische weitere organische Lösungsmittel, die in Mischung mit dem Lösungsmittel L1 eingesetzt werden können, sind z.B.:

- Alkanole mit wenigstens 5 C-Atomen, insbesondere 5 bis 12 C-Atomen wie Amylalkohol, Isoamylalkohol, Hexanole wie n-Hexanol, 2-Ethyl-1-butanol, 4-Methyl-2-pentanol, 2-Ethylhexanol, Isononanol, n-Nonanol, techn. Gemische isomerer Nonylalkohole, 2-Propylheptanol, Isotridecanol, technische Gemisch isomerer Isotridecanole, und dergleichen;
- Cycloalkanole mit wenigstens 5 C-Atomen insbesondere mit 5 bis 12 C-Atomen wie Cyclopentanol, Cyclohexanol, Cycloheptanol, 2-, 3- und 4-Methylcyclohexanol, 3,3,5-Trimethylcyclohexanol und dergleichen;
- aliphatische und cycloaliphatischen Ketone mit 3 bis 12 C-Atomen wie Aceton, Methylethylketon, Diethylketon, Methylpropylketon, Methylbutylketon, Methylisobutylketon, Cyclohexanon, Methylcyclohexanon, Dimethylcyclohexanon, 3,3,5-Trimethylcyclohexanon, Isophoron und dergleichen;
- $C_1$-$C_8$-Alkylester und $C_5$-$C_{10}$-Cycloalkylester aliphatischer $C_1$-$C_4$-Carbonsäuren, insbesondere der Essigsäure wie Methylacetat, Ethylacetat, n-Propylacetat, n-Butylacetat, Isobutylacetat, sec.-Butylacetat, n-Amylacetat, Isoamylacetat, Hexylacetat, 2-Ethylhexylacetat, Octylacetat, Cyclohexylacetat, 2-Butoxyethylacetat, sowie die entsprechenden Propionate und Butyrate;
- Diole mit 2 bis 8 C-Atomen insbesondere Glykol, Propandiol, Butandiol, Hexandiol, 2-Ethylhexan-1,3-diol und 2,4-Diethyloktandiol-1,5;
- N-Di-$C_1$-$C_4$-Alkylamide von aliphatischen Carbonsäuren und $C_1$-$C_4$-Alkyllactame, wie N,N-Dimethylformamid, N,N-Dimethylacetamid, N-Methylpyrrolidon, N-Ethylpyrrolidon und dergleichen; sowie
- aromatischen Kohlenwasserstoffe, insbesondere Mono- oder Di-$C_1$-$C_4$-Alkylsubstituiertes Benzol, speziell Toluol und Xylole.

Der Anteil der von L1 und insbesondere von $C_1$-$C_4$-Alkanolen verschiedenen Lösungsmitteln wird vorzugsweise 30 Vol.-%, insbesondere 20 Vol.-%, besonders bevorzugt 10 Vol.-%, und speziell 5 Vol.-%, bezogen auf die Gesamtmenge des zum Lösen von Pyraclostrobin verwendeten Lösungsmittels nicht überschreiten.

Insbesondere setzt man in Verfahren IVa zum Lösen des Pyraclostrobins in Schritt i) Methanol, Ethanol oder ein Gemisch organischer Lösungsmittel ein, das wenigstens 70 Vol.-%, insbesondere wenigstens 80 Vol.-% und speziell wenigstens 90 Vol.-% wenigstens eines unter Methanol und Ethanol ausgewählten $C_1$-$C_4$-Alkanols enthält.

Neben den vorgenannten organischen Lösungsmitteln kann das zum Lösen des Pyraclostrobins verwendete organische Lösungsmittel bis zu 30 Vol.-%, vorzugsweise nicht mehr als 20 Vol.-%, insbesondere nicht mehr als 10 Vol.-% oder nicht mehr als 5 Vol.-%, z.B. 0,1 bis 20 Vol.-% oder 0,1 bis 10 Vol.-% und speziell 0,2 bis 5 Vol.-% Wasser enthalten.

Zum Lösen der von der Modifikation IV verschiedenen Form des Pyraclostrobins wird man üblicherweise das Pyraclostrobin als feinteiligen Feststoff oder als Schmelze unter Durchmischen in das Lösungsmittel bei einer Temperatur einarbeiten, bei der das Lösungsmittel bzw. Lösungsmittelgemisch das Pyraclostrobin vollständig zu Lösen vermag. In einer bevorzugten Ausführungsform der Erfindung erfolgt das Lösen des Pyraclostrobins bei erhöhter Temperatur, insbesondere bei wenigstens 50°C, speziell bei wenigstens 55°C, wobei die zum Lösen angewendete Temperatur naturgemäß den Siedepunkt des Lösungsmittels nicht überschreiten wird. Häufig wendet man zum Lösen Temperaturen im Bereich von 50 bis 100°C, insbesondere im Bereich von 55 bis 90°C und besonders bevorzugt im Bereich von 60 bis 80°C an. Die Menge an Pyraclostrobin, die im Lösungsmittel L1 gelöst wird, hängt naturgemäß von der Art des Lösungsmittels L1 und der Lösungstemperatur ab und liegt häufig im Bereich von 100 bis 800 g/L, insbesondere im Bereich von 120 bis 700 g/L. Geeignete Bedingungen können vom Fachmann durch Routineexperimente ermittelt werden.

Anschließend bewirkt man die Kristallisation des Pyraclostrobins. Die Kristallisation kann in üblicher Weise, z.B. durch Abkühlen der in Schritt i) erhaltenen Lösung, durch Zugabe eines die Löslichkeit vermindernden Lösungsmittels, insbesondere durch Zugabe von Wasser, oder durch Einengen der Lösung oder durch eine Kombination der vorgenannten Maßnahmen erreichen.

Um eine möglichst vollständige Umwandlung in die Modifikation IV zu erzielen, führt man die Kristallisation über einen Zeitraum (Kristallisationsdauer) von wenigstens 15 h, insbesondere wenigstens 20 h und/oder in Gegenwart von Impfkristallen der Modifikation IV durch. Unter der Kristallisationsdauer versteht der Fachmann die Zeitspanne zwischen Beginn der Maßnahme, welche die Kristallisation bewirkt, und der Isolierung des Pyraclostrobins durch Abtrennung der Kristallmasse von der Mutterlauge.

Die Kristallisation wird in der Regel soweit geführt, dass wenigstens 80 %, vorzugsweise wenigstens 90 %, insbesondere wenigstens 95 Gew.-%, z.B. 95 bis 99,8 Gew.-% des eingesetzten Pyraclostrobins auskristallisieren.

Sofern man bei der Kristallisation Impfkristalle zusetzt, beträgt ihre Menge typischerweise 0,001 bis 10 Gew.-%, häufig 0,005 bis 5 Gew.-%, insbesondere 0,01 bis 1 Gew.-% und speziell 0,05 bis 0,5 Gew.-%, bezogen auf das gelöste Pyraclostrobin. Die Kristallisationsdauer beträgt dann typischerweise wenigstens 2 h, insbesondere wenigstens 4 h und speziell wenigstens 5 h und kann aber auch über einen längeren Zeitraum bis hin zu mehreren Tagen, z.B. 2 bis 3 Tagen erfolgen. Häufig wird die Kristallisationsdauer jedoch 24 h und speziell 14 h nicht überschreiten. Dementsprechend erfolgt die Kristallisation in der Regel über einen Zeitraum von 2 h bis 2 d, häufig 4 bis 24 h und insbesondere von 5 h bis 14 h.

Sofern man die Kristallisation der Modifikation IV in Abwesenheit von Impfkristallen durchführt, beträgt die Kristallisationsdauer in der Regel wenigstens 10 h, insbesondere wenigstens 15 h, speziell wenigstens 20 h und wird in der Regel 21 d häufig 7 d nicht überschreiten.

In einer bevorzugten Ausführungsform der Erfindung wird man so vorgehen, dass man das Pyraclostrobin bei erhöhter Temperatur, vorzugsweise bei wenigstens 50°C, z.B. 50 bis 100°C, vorzugsweise 55 bis 90°C und besonders bevorzugt im Bereich von 60 bis 80°C löst und anschließend die Kristallisation des Pyraclostrobins durch Abkühlen der Lösung bewirkt. Vorzugsweise kühlt man die Lösung des Pyraclostrobins um wenigstens 20 K, insbesondere um 30 bis 50 K ab, um die Kristallisation zu einzuleiten. Der Vorgang des Abkühlens kann in kontrollierter Weise durchgeführt werden, d.h. man kühlt mit einer geringen Abkühlrate von in der Regel nicht mehr als 20 K/h, z.B. 0,5 bis 20 K/h und häufig 1 bis 15 K/h ab. Vorteilhafterweise wird das kontrollierte Abkühlen mit Beginn der Kristallisation durchgeführt. Man kann aber auch rascher abkühlen und man wird dann das Kristallisat über einen längeren Zeitraum in der Mutterlauge bewegen, d.h. bis die gewünschte Kristallisationsdauer erreicht ist, bevor man es isoliert.

Sofern man die Kristallisation in Gegenwart von Impfkristallen der Modifikation IV durchführt, werden diese vorzugsweise erst bei einer Temperatur zugegeben, bei der die Sättigungskonzentration des Pyraclostrobins in dem jeweiligen Lösungsmittel erreicht ist, d.h. bei oder unterhalb derjenigen Temperatur, bei der die gelöste Menge an Pyraclostrobin in dem jeweiligen Lösungsmittel eine gesättigte Lösung bildet. Die Temperaturabhängigkeit der Sättigungskonzentration in einem Lösungsmittel kann der Fachmann in Routineexperimenten ermitteln. Häufig erfolgt die Zugabe der Impfkristalle, wenn die Temperatur der Lösung nicht mehr als 50°C beträgt und insbesondere nicht mehr als 40°C. Vorzugsweise lässt man nach der Zugabe der Impfkristalle die Lösung auf Temperaturen unterhalb 30°C, insbesondere von 25°C oder darunter, z.B. auf Temperaturen im Bereich von 5°C bis 25°C abkühlen, bevor man zur Isolierung der Modifikation IV des Pyraclostrobins das erhaltene kristalline Material von der Mutterlauge abtrennt. Das Abkühlen bei Anwesenheit von Impfkristallen kann kontrolliert mit einer Abkühlrate von in der Regel nicht mehr als 30 K/h z.B. 1 bis 30 K/h, häufig 2 bis 20 K/h und insbesondere 3 bis 15 K/h oder nicht kontrolliert erfolgen.

Es hat sich bewährt, wenn man das kristalline Material noch eine Zeit lang bei Temperaturen unterhalb der Kristallisationstemperatur, z.B. im Bereich von 10 bis 35°C in der Mutterlauge bewegt, z.B. 1 h bis 124 h oder 2 h bis 96 h, um eine vollständige Umwandlung in die Modifikation IV zu gewährleisten. Die Gesamtdauer vom Beginn des Abkühlens bis zur Isolierung der Kristalle durch Abtrennung der Mutterlauge liegt dann in den oben genannten Bereichen.

In einer besonders bevorzugten Ausführungsform des Verfahrens IVa geht man so vor, dass man zunächst das Pyraclostrobin in dem oben genannten Lösungsmittel, insbesondere in einem Lösungsmittel bzw. Lösungsmittelgemisch, dass wenigstens 70 Vol.-%, häufig wenigstens 80 Vol.-%, insbesondere wenigstens 90 und speziell wenigstens 95 Vol.-% wenigstens eines unter Methanol und Ethanol ausgewählten $C_1$-$C_4$-Alkanols enthält, bei erhöhter Temperatur in den oben genannten Temperaturbereichen, insbesondere bei > 50 bis 90 °C und speziell im Bereich von 60 bis 80°C löst, anschließend die Lösung abkühlt, vorzugsweise auf eine Temperatur im Bereich von 20 bis 50°C und insbesondere auf 30 bis 40°C. Vorzugsweise erfolgt das Abkühlen über einen längeren Zeitraum, z.B. über einen Zeitraum von 2 bis 24 h, häufig 4 bis 20 h mit einer Abkühlrate von vorzugsweise 1 K/h bis 20 K/h und insbesondere 3 bis 15 K/h. Zu der so abgekühlten Lösung gibt man dann Impfkristalle der Modifikation IV zu. Anschließend kühlt man dann weiter um wenigstens 5 K und insbesondere um wenigstens 10 K, z.B. um 5 bis 40 K und insbesondere um 10 bis 30 K, z.B. auf Temperaturen von 0 bis 40°C und insbesondere auf von 5 bis 30°C ab. Das zweite Abkühlen erfolgt vorzugsweise über einen Zeitraum von 1 bis 10 h, insbesondere 2 bis 6 h und vorteilhafterweise mit einer Abkühlrate von 2 bis 20 K/h und insbesondere von 3 bis 15 K/h. Hierbei tritt eine Kristallisation des Pyraclostrobins ein.

Alternativ kann man die Kristallisation auch durch Zugabe von Wasser, z.B. von 5 bis 60 Vol.-%, insbesondere 20 bis 55 Vol.-% und speziell von 30 bis 50 Vol.-%, bezogen auf das Volumen des zum Lösen des Pyraclostrobins verwendeten Lösungsmittels bzw. Lösungsmittelgemischs bewirken. Vorzugsweise erfolgt die Zugabe von Wasser über einen längeren Zeitraum, z.B. über einen Zeitraum von 30 min bis 10 h, insbesondere über einen Zeitraum von 1 h bis 8 h. Insbesondere wird man die Zugabe von Wasser und den Zusatz von Impfkristallen miteinander kombinieren. Die Zugabe des Wassers kann in Form von reinem Wasser oder in Form eines Gemisches von Wasser mit einem der vorgenannten Lösungsmittel L1 oder in Mischung mit einem organischen Lösungsmittelgemisch, das überwiegend, d.h. wenigstens 70 Vol.-% L1, bezogen auf das organische Lösungsmittel, enthält, insbesondere im Gemisch mit dem zum Lösen eingesetzten Lösungsmittel zugeführt werden. In letzterem Fall liegt der Anteil an organischem Lösungsmittel in der zugeführten, wasserhaltigen Mischung typischerweise im Bereich von 10 bis 70 Vol.-%, insbesondere 20 bis 60 Vol.-% und speziell 40 bis 50 Vol.-%.

In besonders vorteilhafter Weise bewirkt man die Kristallisation des Pyraclostrobins durch Kombination von Abkühlen und Zugabe von Wasser. Insbesondere geht man so vor, dass man zunächst eine Kristallisation des Pyraclostrobins durch Abkühlen, vorzugsweise unter Zusatz von Impfkristallen, in der oben beschriebenen Weise bewirkt und anschließend die Kristallisation des Pyraclostrobins durch Zugabe von Wasser in den oben genannten Mengen vervollständigt. Insbesondere wird man das Wasser bei einer Temperatur zugeben, bei der bereits ein Teil des in der Lösung enthaltenen Pyraclostrobins, z.B. 5 bis 90 Gew.-% und insbesondere 10 bis 80 Gew.-% bereits auskristallisiert ist. Insbesondere erfolgt die Zugabe des Wassers bei Temperaturen im Bereich von 5 bis 40°C und speziell im Bereich von 10 bis 30°C. Insbesondere wird man soviel Wasser zugeben, dass die Menge an Wasser, bezogen auf die Gesamtmenge an Lösungsmittel + Wasser im Bereich von 20 bis 55 Vol.-% und speziell von 30 bis 50 Vol.% liegt. Insbesondere erfolgt die Zugabe von Wasser über einen Zeitraum von 30 min bis 8 h und besonders bevorzugt über einen Zeitraum von 1 h bis 5 h.

Das Isolieren der Modifikation IV erfolgt nach üblichen Techniken der Trennung fester Bestandteile von Flüssigkeiten, z.B. durch Filtration, Zentrifugation oder durch Abdekantieren. In der Regel wird man den isolierten Feststoff waschen, z.B. mit dem zur Kristallisation verwendeten Lösungsmittel, mit Wasser oder mit einem Gemisch aus dem zur Kristallisation verwendeten organischen Lösungsmittel mit Wasser. Das Waschen kann in ein oder mehreren Schritten erfolgen, wobei man häufig im letzten Waschschritt mit Wasser wäscht. Das Waschen erfolgt typischerweise bei Temperaturen unterhalb 30°C, häufig unterhalb 25°C und insbesondere unterhalb 20°C, um den Verlust an Wertprodukt möglichst gering zu halten. Anschließend kann man die erhaltene Modifikation IV trocknen und dann der Weiterverarbeitung zuführen. Häufig wird man jedoch den nach Waschen erhaltenen feuchten Wirkstoff, insbesondere einen wasserfeuchten Wirkstoff der weiteren Verarbeitung zuführen.

In einem anderen Herstellungsverfahren der kristallinen Modifikation IV des Pyraclostrobins (im Folgenden auch Verfahren IVb) führt man die folgenden Schritte durch:

i) Herstellung einer Suspension einer von Modifikation IV verschiedenen Form des Pyraclostrobins in einem organischer Lösungsmittel (Lösungsmittel L2);
ii) gegebenenfalls Zugabe von Impfkristallen der Modifikation IV zu der Suspension;
iii) Bewegen der Suspension, bis das Pyraclostrobin zu wenigstens 90 % in Form der Modifikation IV vorliegt.

In dem Verfahren IVb können grundsätzlich zur Herstellung der Suspension alle der für das Verfahren IVa genannten von Modifikation IV verschiedenen Form des Pyraclostrobins eingesetzt werden. Bezüglich der Reinheit gilt das für Verfahren IVa Gesagte.

Bei dem Lösungsmittel L2 handelt es sich typischerweise um ein organisches Lösungsmittel oder Lösungsmittelgemisch, das Pyraclostrobin zumindest teilweise gegebenenfalls bei erhöhter Temperatur zu lösen vermag. Insbesondere handelt es sich um ein organisches Lösungsmittel oder Lösungsmittelgemisch, in welchem Pyraclostrobin bei einer Temperatur von 40°C eine Löslichkeit von wenigstens 100 g/L und vorteilhafterweise nicht mehr als 800 g/L, insbesondere nicht mehr als 700 g/L aufweist.

Beispiele für geeignete Lösungsmittel L2 umfassen die unter den Lösungsmitteln L1 genannten $C_1$-$C_4$-Alkanole, Alkanole mit 5 bis 12 C-Atomen, Cycloalkanole mit 5 bis 12 C-Atomen, aliphatische und cycloaliphatischen Ketone mit 3 bis 12 C-Atomen, $C_1$-$C_8$-Alkylester und $C_5$-$C_{10}$-Cycloalkylester aliphatischer $C_1$-$C_4$-Carbonsäuren, insbesondere der Essigsäure, Diole mit 2 bis 8 C-Atomen, N-Di-$C_1$-$C_4$-Alkylamide von aliphatischen Carbonsäuren und $C_1$-$C_4$-Alkyllactame sowie aromatischen Kohlenwasserstoffe, insbesondere Mono- oder Di-$C_1$-$C_4$-Alkyl-substituiertes Benzol und Mischungen dieser Lösungsmittel.

Vorteilhafterweise enthält das zum Suspendieren des Pyraclostrobins eingesetzte organische Lösungsmittel L2 wenigstens 50 Vol.-%, insbesondere wenigsten 60 Vol.-%, besonders bevorzugt wenigstens 70 Vol.-%, ganz besonders bevorzugt wenigstens 80 Vol.-% und speziell wenigstens 90 Vol.-% wenigstens eines $C_1$-$C_4$-Alkanols, wobei Methanol, Ethanol, n-Propanol und Isopropanol besonders bevorzugt sind. Der Anteil der von $C_1$-$C_4$-Alkanolen verschiedenen Lösungsmitteln wird dementsprechend vorzugsweise 50 Vol.-%, insbesondere 40 Vol.-%, besonders bevorzugt 30 Vol.-%, ganz besonders bevorzugt 20 Vol.-% und speziell 10 Vol.-% nicht überschreiten.

Insbesondere setzt man in Verfahren IVb zum Suspendieren des Pyraclostrobins in Schritt i) Isopropanol, Ethanol oder ein Gemisch organischer Lösungsmittel ein, das wenigstens 70 Vol.-%, insbesondere wenigstens 80 Vol.-%, besonders bevorzugt wenigstens 90 Vol.-% Isopropanol und/oder Ethanol enthält.

Neben den vorgenannten organischen Lösungsmitteln kann das organische Lösungsmittel L2 geringe Mengen, vorzugsweise nicht mehr als 25 Vol.-%, insbesondere nicht mehr als 10 Vol.-% und besonders bevorzugt nicht mehr als 5 Vol.-% Wasser enthalten.

Zur Herstellung der Suspension kann man festes oder geschmolzenes Pyraclostrobin in dem Lösungsmittel L2 in an sich bekannter Weise suspendieren, wobei man die Temperatur des Lösungsmittels L2 und Menge an Pyraclostrobin so wählt, dass das Pyraclostrobin nicht vollständig gelöst wird. Diese Parameter kann ein Fachmann anhand von Routineexperimenten ermitteln. Typischerweise wird man eine Temperatur im Bereich von 20 bis 40°C wählen. Die Menge an Pyraclostrobin, die im Lösungsmittel L2 suspendiert wird, liegt häufig im Bereich von 100 bis 800 g/L, insbesondere im Bereich von 120 bis 700 g/L. Typischerweise wendet man zum Suspendieren des festen oder geschmolzenen Pyraclostrobins Scherkräfte an, z.B. indem man die Suspension mit einem geeigneten Rührer rührt. Geeignete Rührertypen sind dem Fachmann geläufig, z.B. aus M. Zlokarnik, Stirring, in Ullmann's Encyclopedia of Industrial Chemistry, 5th ed. on CD-ROM, Wiley-VCH 1997.

In einer bevorzugten Ausführungsform der Erfindung stellt man die Suspension her, indem man Pyraclostrobin zunächst in dem Lösungsmittel L2 vollständig oder nahezu vollständig löst, in vorteilhafter Weise unter Anwendung erhöhter Temperatur, insbesondere bei Temperaturen im Bereich von 40 bis 80°C, und dann eine partielle Kristallisation des Pyraclostrobins durchführt, typischerweise durch Einengen und/oder durch Temperaturerniedrigung, typischerweise um wenigstens 10 K, insbesondere um wenigstens 20 K, z.B. um 20 bis 50 K.

Die so erhaltene Suspension wird dann, gegebenenfalls unter Zusatz von Impfkristallen der Modifikation IV solange bewegt, bis die Umwandlung in die Modifikation vollständig ist, d.h. der Gehalt an Modifikation IV in dem suspendierten Feststoff wenigstens 90 Gew.-% beträgt. Die hierfür erforderliche Zeit kann der Fachmann durch Probennahme und Analyse des Materials mittels Röntgenpulverdiffraktometrie (XRD) oder DSC routinemäßig ermitteln.

Sofern man die Umwandlung unter Zusatz von Impfkristallen durchführt, wird man die Suspension typischerweise 12 bis 48 h, insbesondere 14 bis 36 h bewegen, um die gewünschte Umwandlung zu erreichen, wobei längere Zeiten nicht nachteilig sind. Bezüglich der Menge an Impfkristallen gilt das zuvor für Verfahren IVa Gesagte analog. Die Zugabe der Impfkristalle erfolgt typischerweise zu der Suspension bei Temperaturen im Bereich von 20 bis 40°C. Sofern man ohne Impfkristalle arbeitet, wird man die Suspension vorzugsweise wenigstens 24 h, insbesondere wenigstens 48 h und besonders bevorzugt wenigstens 72 h bewegen, bevor man das kristalline Material von der Mutterlauge abtrennt.

Die Temperatur der Suspension liegt vorteilhafterweise im Bereich von 20 bis 40°C. Das Bewegen erfolgt typischerweise durch Rühren.

Die Isolierung der Modifikation IV aus der Suspension und die weitere Verarbeitung kann auf die für das Verfahren IVa beschriebene Weise erfolgen.

Durch die erfindungsgemäßen Verfahren IVa und IVb wird die kristalline Modifikation IV mit einem Gehalt an Pyraclostrobin von wenigstens 98 Gew.-%, insbesondere wenigstens 99 Gew.-% erhalten. Der Anteil an Modifikation IV, bezogen auf die Gesamtmenge an Pyraclostrobin liegt typischerweise bei wenigstens 90 %, häufig wenigstens 95 % und insbesondere wenigstens 98 %.

Im Zusammenhang mit den Untersuchungen zu der kristallinen Modifikation IV wurden drei weitere kristalline Modifikationen des Pyraclostrobins (Modifikationen I, II und III) gefunden. Die Modifikationen I, II und III des Pyraclostrobins sind thermodynamisch stabiler als amorphes Pyraclostrobin jedoch gegenüber der Modifikation IV nur metastabil und wandeln sich unter bestimmten Bedingungen in die Modifikation IV um. Bezüglich der Stabilität gilt: Stabilität (Modifikation I) < Stabilität (Modifikation II) < Stabilität (Modifikation III) < Stabilität (Modifikation IV). Die Modifikationen I, II, III und IV bilden ein monotropes Phasensystem (Schmelzenthalpie).

Die kristalline Modifikation I des Pyraclostrobins zeigt in einem Röntgenpulverdiffraktogramm bei 25°C wenigstens vier, insbesondere wenigstens fünf, häufig wenigstens sechs und speziell alle der folgenden Reflexe:

$$d = 6,57 \pm 0,01 \text{ Å}$$
$$d = 5,80 \pm 0,01 \text{ Å}$$
$$d = 4,78 \pm 0,01 \text{ Å}$$
$$d = 4,22 \pm 0,01 \text{ Å.}$$
$$d = 3,96 \pm 0,01 \text{ Å.}$$
$$d = 3,52 \pm 0,01 \text{ Å.}$$
$$d = 3,42 \pm 0,01 \text{ Å.}$$
$$d = 3,34 \pm 0,01 \text{ Å.}$$

Kristallines Pyraclostrobin der Modifikation I weist typischerweise einen Schmelzpunkt im Bereich von 55 bis 56°C auf. Die Schmelzwärme, d.h. die Energiemenge, die zum Schmelzen der kristallinen Modifikation I benötigt wird, beträgt etwa 63 bis 66 J/g und insbesondre etwa 65 ± 1 J/g.

Die Modifikation I des Pyraclostrobins fällt typischerweise beim Abkühlen einer Pyrac-lostrobin-Schmelze an, wenn die Reinheit des zur Herstellung der Schmelze eingesetzten Pyraclostrobins wenigstens 95 % beträgt. Die Kristallisation der Modifikation I kann durch Tempern des Materials bei Temperaturen im Bereich von 40 bis 50°C beschleunigt werden. Vorzugsweise wird man das Tempern jedoch nicht länger als 14 Tage durchführen, da ansonsten die Umwandlung in die stabileren Modifikationen II und III eintritt.

Die kristalline Modifikation II des Pyraclostrobins zeigt in einem Röntgenpulverdiffraktogramm bei 25°C wenigstens vier, typischerweise wenigstens fünf, häufig wenigstens sechs, insbesondere wenigstens 7 und speziell alle der folgenden Reflexe:

$$d = 5,93 \pm 0,01 \text{ Å}$$
$$d = 5,82 \pm 0,01 \text{ Å}$$
$$d = 4,89 \pm 0,01 \text{ Å}$$
$$d = 4,78 \pm 0,01 \text{ Å}$$
$$d = 4,71 \pm 0,01 \text{ Å.}$$
$$d = 3,97 \pm 0,01 \text{ Å.}$$
$$d = 3,89 \pm 0,01 \text{ Å.}$$
$$d = 3,77 \pm 0,01 \text{ Å.}$$
$$d = 3,75 \pm 0,01 \text{ Å.}$$
$$d = 3,57 \pm 0,01 \text{ Å.}$$
$$d = 3,43 \pm 0,01 \text{ Å.}$$

Kristallines Pyraclostrobin der Modifikation II weist typischerweise einen Schmelzpunkt im Bereich von 57 bis 58°C auf. Die Schmelzwärme, d.h. die Energiemenge, die zum Schmelzen der kristallinen Modifikation II benötigt wird, beträgt etwa 67 bis 70 J/g und insbesondere etwa 69 $\pm$ 1 J/g.

Untersuchungen an Einkristallen der Modifikation II zeigen, dass die zugrunde liegende Kristallstruktur triklin ist und die Raumgruppe P-1 aufweist. Die charakteristischen Daten der Kristallstruktur von Modifikation II sind in der Tabelle 2 angegeben:

Tabelle 2: Kristallographische Daten der Modifikation II

| Parameter | Modifikation IV |
|---|---|
| Klasse | Triklin |
| Raumgruppe | P-1 |
| a | 789,69(16) pm |
| b | 1012,40(14) pm |
| c | 1228,9(2) pm |
| $\alpha$ | 96,733(10) ° |
| $\beta$ | 99,833(14) ° |
| $\gamma$ | 105,405(12) ° |
| Volumen | 0,9194(3) $nm^3$ |
| Z | 2 |
| Dichte (berechnet) | 1,401 $mg/m^3$ |
| R1, wR2 | 0,0606, 0,1414 |
| a,b,c = Kantenlänge der Elementarzelle $\alpha$,$\beta$,$\gamma$ = Winkel der Elementarzelle Z = Anzahl der Moleküle in der Elementarzelle | |

Die Herstellung der kristallinen Modifikation II des Pyraclostrobins gelingt durch ein Verfahren, das die folgenden Schritte umfasst:

i) Lösen von amorphem Pyraclostrobin in einem organischen Lösungsmittel, das wenigstens 50 Vol.-% wenigstens eines $C_1$-$C_4$-Alkanols und vorzugsweise nicht mehr als 30 Vol.-%, insbesondere nicht mehr als 10 Vol.-% Wasser enthält; und
ii) Bewirken einer Kristallisation von Pyraclostrobin über einen Zeitraum von weniger als 10 h in Abwesenheit von Impfkristallen der Modifikation IV.

Bezüglich des Lösens des Pyraclostrobins, insbesondere der zu verwendenden Lösungsmittel, der Temperaturen, Konzentrationen etc. gilt das für Verfahren IVa Gesagte in analoger Weise.

Das Bewirken der Kristallisation kann grundsätzlich analog den für das Verfahren IVa geschilderten Methoden erfolgen. Typischerweise wird man die Kristallisation durch Abkühlen der Lösung um wenigstens 20 K, insbesondere um 30 bis 60 K bewirken.

Im Unterschied zu den Verfahren IVa und IVb beträgt die Gesamtdauer des Kristallisationsvorgangs, d.h. die Zeitspanne zwischen Beginn der Maßnahme, welche die Kristallisation bewirkt, und der Isolierung des Pyraclostrobins durch Abtrennung von der Mutterlauge, weniger als 10 h, insbesondere 2 h bis 8 h.

Gegebenenfalls wird man die Kristallisation in Gegenwart von Impfkristallen der Modifikation II durchführen. Die Menge an Impfkristallen beträgt dann typischerweise 0,01 bis 10 Gew.-%, häufig 0,02 bis 5 Gew.-%, insbesondere 0,03 bis 1 Gew.-% und speziell 0,05 bis 0,5 Gew.-%, bezogen auf das gelöste Pyraclostrobin. Die Zugabe der Impfkristalle erfolgt typischerweise während der Kristallisation der Modifikation II und insbesondere zu Beginn der Kristallisation der Modifikation II und vorzugsweise bei oder unterhalb der Temperatur, bei der die Sättigungskonzentration an Pyraclostrobin in dem jeweiligen Lösungsmittel erreicht ist.

Auf diese Weise kann die Modifikation II mit einem Gehalt an Pyraclostrobin von wenigstens 98 Gew.-%, insbesondere wenigstens 99 Gew.-% hergestellt werden. Der Anteil an Modifikation II in dem so hergestellten kristallinen Pyraclostrobin beträgt in der Regel wenigstens 90 %.

Die kristalline Modifikation III des Pyraclostrobins zeigt in einem Röntgenpulverdiffraktogramm bei 25°C wenigstens drei, insbesondere wenigstens vier, häufig wenigstens fünf und speziell alle der folgenden Reflexe:

$$d = 5,36 \pm 0,01 \text{ Å}$$
$$d = 5,39 \pm 0,01 \text{ Å}$$
$$d = 4,31 \pm 0,01 \text{ Å}$$
$$d = 3,68 \pm 0,01 \text{ Å}.$$
$$d = 3,29 \pm 0,01 \text{ Å}.$$
$$d = 2,82 \pm 0,01 \text{ Å}.$$

Kristallines Pyraclostrobin der Modifikation III weist typischerweise einen Schmelzpunkt im Bereich von 59 bis 60°C auf. Die Schmelzwärme, d.h. die Energiemenge, die zum Schmelzen der kristallinen Modifikation I benötigt wird, beträgt etwa 69 bis 72 J/g und insbesondre etwa 71 $\pm$ 1 J/g.

Die Herstellung der Modifikation III gelingt ähnlich der Herstellung der Modifikation I durch Kristallisation einer Pyraclostrobin-Schmelze, wobei man im Unterschied zur Herstellung der Modifikation I die Schmelze über einen längeren Zeitraum bei Temperaturen im Bereich von 18 bis 25°C (Umgebungstemperatur) belässt, bis im XRD die Bildung der Modifikation erkennbar ist. Die Modifikation kann bei niedrigen Temperaturen, vorzugsweise unterhalb -15°C, z.B. im Bereich von -18 bis -30°C über einen längeren Zeitraum aufbewahrt werden.

Wie bereits zuvor erwähnt, eignen sich die Modifikationen II und IV und insbesondere die Modifikation IV zur Herstellung von Pflanzenschutzmitteln und insbesondere zur Herstellung wässriger Suspensionskonzentrate. Dementsprechend ist ein weiterer Gegenstand der Erfindung ein Mittel für den Pflanzenschutz, enthaltend Pyraclostrobin in Form der Modifikation IV oder in Form der Modifikation II, gegebenenfalls eine flüssige Phase sowie gegebenenfalls übliche, in der Regel feste Trägerstoffe und/oder Hilfsmittel.

Als Trägerstoffe kommen grundsätzlich alle festen Substanzen in Betracht, die üblicherweise in Pflanzenschutzmitteln, insbesondere in Fungiziden zum Einsatz kommen. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Im Falle von flüssigen Formulierungen der Modifikationen II und/oder IV weisen die Zusammensetzungen eine flüssige Phase auf. Als flüssige Phase kommen grundsätzlich Wasser sowie solche organischen Lösungsmittel in Betracht, in denen Pyraclostrobin eine geringe oder keine Löslichkeit besitzt, z.B. solche, in denen die Löslichkeit von Pyraclostrobin bei 25°C und 1013 mbar nicht mehr als 1 Gew.-%, insbesondere nicht mehr als 0,1 Gew.-% und speziell nicht mehr als 0,01 Gew.-% beträgt.

Typische Hilfsmittel umfassen oberflächenaktive Substanzen, insbesondere die in Pflanzenschutzmitteln üblicherweise eingesetzten Netzmittel und Dispergier(hilfs)mittel, weiterhin die Viskosität verändernde Additive (Verdicker, Andicker), Antischaummittel, Frostschutzmittel, Mittel zur Einstellung des pH-Wertes, Stabilisatoren, Anticaking-Mittel und Biozide (Konservierungsmittel).

Die Erfindung betrifft insbesondere Mittel für den Pflanzenschutz in Form eines wässrigen Suspensionskonzentrats (SC). Derartige Suspensionskonzentrate enthalten das Pyraclostrobin der Modifikation II und/oder IV in einer feinteiligen partikulären Form, wobei die Pyraclostrobin-Partikel in einem wässrigen Medium suspendiert vorliegen. Die Größe der Wirkstoffpartikel, d.h. die Größe, welche 90 Gew.-% der Wirkstoffpartikel nicht überschreiten, liegt dabei typischerweise unterhalb 30 $\mu$m, insbesondere unterhalb 20 $\mu$m. Vorteilhafterweise weisen in den erfindungsgemäßen SC's wenigstens 40 Gew.-% und insbesondere wenigstens 60 Gew.-% der Teilchen Durchmesser unterhalb 2 $\mu$m auf.

Suspensionskonzentrate enthalten neben dem Wirkstoff typischerweise oberflächenaktive Substanzen, sowie gegebenenfalls Antischaummittel, Verdicker (= Andicker), Frostschutzmittel, Stabilisatoren (Biozide), Mittel zur Einstellung des pH-Wertes und Anticaking-Mittel.

Die Menge an Wirkstoff, d.h. die Gesamtmenge an Pyraclostrobin der Modifikation II und/oder IV sowie an gegebenenfalls weiteren Wirkstoffen, liegt in derartigen SC's üblicherweise im Bereich von 10 bis 70 Gew.-%, insbesondere im Bereich von 20 bis 50 Gew.-%, bezogen auf das Gesamtgewicht des Suspensionskonzentrats.

Als oberflächenaktive Substanzen kommen vorzugsweise anionische und nichtionische Tenside in Betracht. Geeignete oberflächenaktive Substanzen sind auch Schutzkolloide. Die Menge an oberflächenaktiven Substanzen wird in der Regel 0,5 bis 20 Gew.-%, insbesondere 1 bis 15 Gew.-% und besonders bevorzugt 1 bis 10 Gew.-%, bezogen auf das Gesamtgewicht der erfindungsgemäßen SC's betragen. Vorzugsweise umfassen die die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 10:1 bis 1:10 liegt.

Zu den Beispielen anionischer oberflächenaktiver Substanzen (Tenside) zählen Alkylarylsulfonate, Phenylsulfonate, Alkylsulfate, Alkylsulfonate, Alkylethersulfate, Alkylarylethersulfate, Alkylpolyglykoletherphosphate, Polyarylphenyletherphosphate, Alkylsulfosuccinate, Olefinsulfonate, Paraffinsulfonate, Petroleumsulfonate, Tauride, Sarkoside, Fettsäuren, Alkylnaphthalinsulfonsäuren, Naphthalinsulfonsäuren, Ligninsulfonsäuren, Kondensationsprodukte sulfonierter Naphthaline mit Formaldehyd oder mit Formaldehyd und Phenol und gegebenenfalls Harnstoff sowie Kondensationsprodukte aus Phenolsulfonsäure, Formaldehyd und Harnstoff, Lignin-Sulfit-Ablauge und Ligninsulfonate, Alkylphosphate, Alkylarylphosphate, z.B. Tristyrylphosphate, sowie Polycarboxylate wie z.B. Polyacrylate, Maleinsäureanhydrid/Olefin-Copolymere (z.Bsp. Sokalan® CP9, BASF), einschließlich der Alkali-, Erdalkali-, Ammonium- und Amin-Salze der vorgenannten Substanzen. Bevorzugte anionische oberflächenaktive Substanzen sind solche, die wenigstens eine Sulfonatgruppe tragen und insbesondere deren Alkalimetall- und deren Ammoniumsalze.

Beispiele für nichtionische Tenside umfassen Alkylphenolalkoxylate, Alkoholalkoxylate, Fettaminalkoxylate, Polyoxyeethylenglycerolfettsäureester, Rizinusölalkoxylate, Fettsäurealkoxylate, Fettsäureamidalkoxylate, Fettsäurepolydiethanolamide, Lanolinethoxylate, Fettsäurepolyglykolester, Isotridecylalkohol, Fettsäureamide, Methylcellulose, Fettsäureester, Alkylpolyglykoside, Glycerolfettsäureester, Polyethylenglykol, Polypropylenglykol, Polyethylenglykolpolypropylenglykol-Blockcopolymere, Polyethylenglykolalkylether, Polypropylenglykolalkylether, Polyethylenglykolpolypropylenglykolether-Blockcopolymere (Polyethylenoxid-Polypropylenoxid-Blockcopolymere) und deren Gemische. Bevorzugte nichtionische oberflächenaktive Substanzen sind Fettalkoholethoxylate, Alkylpolyglykoside, Glycerolfettsäureester, Rizinusölalkoxylate, Fettsäurealkoxylate, Fettsäureamidalkoxylate, Lanolinethoxylate, Fettsäurepolyglykolester und Ethylenoxid-Propylenoxid-Blockcopolymere und deren Mischungen.

Schutzkolloide sind typischerweise wasserlösliche, amphiphile Polymere. Beispiele hierfür sind Proteine und denaturierte Proteine wie Casein, Polysaccharide wie wasserlösliche Stärkederivate und Cellulosederivate, insbesondere hydrophob modifizierte Stärken und Cellulosen, weiterhin Polycarboxylate wie Polyacrylsäure und Acrylsäurecopolymere, Polyvinylalkohol, Polyvinylpyrrolidon, Vinylpyrrolidon-Copolymere, Polyvinylamine, Polyethylenimine, und Polyalkylenether.

Insbesondere umfassen die erfindungsgemäßen SC's wenigstens eine oberflächenaktive Substanz, welche die Benetzung der Pflanzenteile durch die wässrige Applikationsform fördert (sog. Netzmittel), und wenigstens eine oberflächenaktive Substanz, welche die Dispersion der Wirkstoffpartikel im SC stabilisiert (Dispergier(hilfs)mittel). Die Menge an Netzmittel liegt typischerweise im Bereich von 0,5 bis 10 Gew.-%, insbesondere 0,5 bis 5 Gew.-% und speziell 0,5 bis 3 Gew.-%, bezogen auf das Gesamtgewicht des SC's. Die Menge an Dispergiermittel beträgt typischerweise 0,5 bis 10 Gew.-% und insbesondere 0,5 bis 5 Gew.-%, bezogen auf das Gesamtgewicht des SC's.

Bevorzugte Netzmittel sind anionischer oder nichtionischer Natur und beispielsweise unter Naphthalinsulfonsäuren einschließlich deren Alkali-, Erdalkali-, Ammonium- und Amin-Salzen, weiterhin Fettalkoholethoxylaten, Alkylpolyglykosiden, Glycerolfettsäureestern, Rizinusölalkoxylaten, Fettsäurealkoxylaten, Fettsäureamidalkoxylaten, Fettsäurepolydiethanolamiden, Lanolinethoxylaten und Fettsäurepolyglykolestern ausgewählt.

Bevorzugte Dispergierhilfsmittel sind anionischer oder nichtionischer Natur und z.B. unter Polyethylenglykolpolypropylenglykol-Blockcopolymeren, Polyethylenglykolalkylether, Polypropylenglykolalkylether, Polyethylenglykolpolypropylenglykolether-Blockcopolymeren, Alkylarylphosphaten, z.B. Tristyrylphosphaten, Ligninsulfonsäuren, Kondensationsprodukten sulfonierter Naphthaline mit Formaldehyd oder mit Formaldehyd und Phenol und gegebenenfalls Harnstoff sowie Kondensationsprodukten aus Phenolsulfonsäure, Formaldehyd und Harnstoff, Lignin-Sulfit-Ablaugen und Ligninsulfonaten, Polycarboxylaten wie z.B. Polyacrylate, Maleinsäureanhydrid/Olefin-Copolymere (z.B. Sokalan® CP9, BASF), einschließlich der Alkali-, Erdalkali-, Ammonium- und Amin-Salze der vorgenannten Substanzen ausgewählt.

Für die erfindungsgemäßen SC's geeignete, die Viskosität verändernde Additive (Andicker) sind insbesondere Verbindungen, die der Formulierung ein pseudoplastisches Fließverhalten verleihen, d.h. hohe Viskosität im Ruhezustand und niedrige Viskosität im bewegten Zustand. Geeignet sind grundsätzlich alle für diesen Zweck in Suspensionskonzentraten eingesetzte Verbindungen. Zu nennen sind beispielsweise anorganische Substanzen wie Bentonite oderAttapulgite (z.B. Attaclay® Firma Engelhardt), und organische Substanzen wie Polysaccharide und Heteropolysaccharide wie Xanthan Gum® (Kelzan® der Fa. Kelco), Rhodopol® 23 (Rhone Poulenc) oder Veegum® (Firma R.T. Vanderbilt) zu nennen, wobei Xanthan-Gum® bevorzugt verwendet wird. Die Menge der die Viskosität verändernden Additive beträgt häufig 0,1 bis 5 Gew.-%, bezogen auf das Gesamtgewicht des SC's.

Als für die erfindungsgemäßen SC's geeignete Antischaummittel kommen beispielsweise für diesen Zweck bekannte Silikonemulsionen (Silikon® SRE, Firma Wacker oder Rhodorsil® der Firma Rhodia), langkettige Alkohole, Fettsäuren, Entschäumer vom Typ wässriger Wachsdispersionen, feste Entschäumer (sog. Compounds), fluororganische Verbindungen und deren Gemische in Betracht. Die Menge an Antischaummittel beträgt typischerweise 0,1 bis 1 Gew.-%, bezogen auf das Gesamtgewicht des SC's.

Den erfindungsgemäßen Suspensionskonzentraten kann man zur Stabilisierung auch Konservierungsmittel zusetzen. Geeignete Konservierungsmittel sind solche auf Basis von Isothiazolonen, beispielsweise Proxel® der Fa. ICI oder Acticide® RS der Fa. Thor Chemie oder Kathon® MK der Firma Rohm & Haas. Die Menge an Konservierungsmittel beträgt typischerweise 0,05 bis 0,5 Gew.-%, bezogen auf das Gesamtgewicht des SC's.

Geeignete Frostschutzmittel sind flüssige Polyole, z.B. Ethylenglycol, Propylenglycol oder Glycerin. Die Menge an Frostschutzmitteln beträgt in der Regel 1 bis 20 Gew.-%, insbesondere 5 bis 10 Gew.-%, bezogen auf das Gesamtgewicht des Suspensionskonzentrats.

Gegebenenfalls können die erfindungsgemäßen SC's Puffer zur pH-Wert Regulation enthalten. Beispiele für Puffer sind Alkalisalze schwacher anorganischer oder organischer Säuren wie z.B. Phosphorsäure, Borsäure, Essigsäure, Propionsäure, Citronensäure, Fumarsäure, Weinsäure, Oxalsäure und Bernsteinsäure.

Sofern die Formulierungen der kristallinen Modifikationen des Pyraclostrobins zur Saatgutbehandlung eingesetzt werden, können sie weitere übliche Bestandteile enthalten, wie sie bei der Saatgutbehandlung, z.B. bei Beizen oder Coaten eingesetzt werden. Hierzu zählen neben den vorgenannten Bestandteilen insbesondere Farbmittel, Kleber, Füllstoffe und Weichmacher.

Als Farbmittel kommen alle die für derartige Zwecke üblichen Farbstoffe und Pigmente in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C. I. Pigment Red 112 und C. I. Solvent Red 1, Pigment blue 15:4, Pigment blue 15:3, Pigment blue 15:2, Pigment blue 15:1, Pigment blue 80, Pigment yellow 1, Pigment yellow 13, Pigment red 48:2, Pigment red 48:1, Pigment red 57:1, Pigment red 53:1, Pigment orange 43, Pigment orange 34, Pigment orange 5, Pigment green 36, Pigment green 7, Pigment white 6, Pigment brown 25, Basic violet 10, Basic violet 49, Acid red 51, Acid red 52, Acid red 14, Acid blue 9, Acid yellow 23, Basic red 10, Basic red 108 bekannten Farbstoffe und Pigmente. Die Menge an Farbmittel wird üblicherweise nicht mehr als 20 Gew.-% der Formulierung ausmachen und liegt vorzugsweise im Bereich von 0,1 bis 15 Gew.-%, bezogen auf das Gesamtgewicht der Formulierung.

Als Kleber kommen alle üblichen in Beizmitteln einsetzbaren Bindemittel in Frage. Beispiele für geeignete Bindemittel umfassen thermoplastische Polymere wie Polyvinylpyrrolidon, Polyvinylacetat, Polyvinylalkohol und Tylose weiterhin Polyacrylate, Polymethacrylate, Polybutene, Polyisobutene, Polystyrol, Polyethylenamine, Polyethylenamide, die vorgenannten Schutzkolloide, Polyester, Polyetherester, Polyanhydride, Polyesterurethane, Polyesteramide, thermoplastische Polysaccharide, z.B. Cellulosederivate wie Celluloseester, Celluloseether, Celluloseetherester, einschließlich Methylcellulose, Ethylcellullose, Hydroxymethylcellulose, Carboxymethylcellulose, Hydroxypropylcellulose und Stärkederivate und modifizierte Stärken, Dextrine, Maltodextrine, Alginate und Chitosane, weiterhin Fette, Öle, Proteine, einschließlich Casein, Gelatine und Zein, Gummi-Arabicum, Schellack. Vorzugsweise sind die Kleber pflanzenverträglich, d.h. sie weisen keine oder keinen nennenswerten phytotoxischen Wirkungen auf. Vorzugsweise sind die Kleber biologisch abbaubar. Vorzugsweise ist der Kleber so gewählt, dass er als Matrix für die aktiven Bestandteile der Formulierung wirkt. Die Menge an Kleber wird üblicherweise nicht mehr als 40 Gew.-% der Formulierung ausmachen und liegt vorzugsweise im Bereich von 1 bis 40 Gew.-% und insbesondere im Bereich von 5 bis 30 Gew.-%, bezogen auf das Gesamtgewicht der Formulierung.

Neben dem Kleber kann die Formulierung auch inerte Füllstoffe enthalten. Beispiele hierfür sind die vorgenannten festen Trägermaterialien, insbesondere feinteilige anorganische Materialien wie Tone, Kreide, Bentonit, Kaolin, Talkum, Perlit, Mica, Kieselgel, Diatomeenerde, Quarzpulver, Montmorillonit, aber auch feinteilige organische Materialen, wie Holzmehl, Getreidemehl, Aktivkohle und dergleichen. Die Menge an Füllstoff wird vorzugsweise so gewählt, dass die Gesamtmenge an Füllstoff 75 Gew.-%, bezogen auf das Gesamtgewicht aller nicht flüchtigen Bestandteile der Formulierung nicht überschreitet. Häufig liegt die Menge an Füllstoff im Bereich von 1 bis 50 Gew.-%, bezogen auf das Gesamtgewicht aller nicht flüchtigen Bestandteile der Formulierung.

Daneben kann die Formulierung noch einen Weichmacher enthalten, der die Flexibilität der Beschichtung erhöht. Beispiele für Weichmacher sind oligomere Polyalkylenglykole, Glycerin, Dialkylphthalate, Alkylbenzylphthalate, Glykolbenzoate und vergleichbare Verbindungen. Die Menge an Weichmacher in der Beschichtung liegt häufig im Bereich von 0,1 bis 20 Gew.-%, bezogen auf das Gesamtgewicht aller nicht flüchtigen Bestandteile der Formulierung.

Pyraclostrobin in Form der Modifikation IV oder in Form der Modifiktation II können in an sich bekannter Weise zur Bekämpfung von pflanzenpathogenen Pilzen eingesetzt werden. Sie können insbesondere gemeinsam mit weiteren Wirkstoffen zur Wirkungssteigerung und/oder zur Verbreiterung des Anwendungsspektrums formuliert werden. Hierzu zählen grundsätzlich alle Insektizide und Fungizide, die typischerweise gemeinsam mit Pyraclostrobin eingesetzt werden. Die neuen Modifikationen des Pyraclostrobins können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Triticale, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Insbesondere eignen sich die Modifikationen II und IV zur gemeinsamen Formulierung als Suspensionskonzentrate mit Wirkstoffen, die ihrerseits als Suspensionskonzentrate formuliert werden können. Dementsprechend betrifft eine bevorzugte Ausführungsform der Erfindung Suspensionskonzentrate, die neben Pyraclostrobin der Modifikation II und/oder Modifikation IV wenigstens einen weiteren Wirkstoff in Form in feinteiliger, partikulärer Form enthalten. Bezüglich der Teilchengrößen, Wirkstoffmenge, Hilfsmittel gilt das zuvor Gesagte.

Typische Mischungspartner des Pyraclostrobins sind beispielsweise:

- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph,
- Anilinopyrimidine wie Pyrimethanil, Mepanipyrim oder Cyprodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinioconazol, Epoxiconazol, Fenbuconazol, Fluquinconazol, Flusilazol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Thiophanat-ethyl,Tiadinil, Tricyclazol, Triforine,
- Nitrophenylderivate, wie Binapacryl, Dinocap, Dinobuton, Nitrophthal-isopropyl,
- Phenylpyrrole wie Fenpiclonil oder Fludioxonil,
- Schwefel,
- Sonstige Fungizide wie Acibenzolar-S-methyl, Benthiavalicarb, Carpropamid, Chlorothalonil, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Toloclofos-methyl, Quintozene, Zoxamid,
- Sulfensäurederivate wie Captafol, Captan, Dichlofluanid, Folpet, Tolylfluanid
- Zimtsäureamide und Analoge wie Dimethomorph, Flumetover oder Flumorph,
- 6-Aryl-[1,2,4]triazolo[1,5-a]pyrimidine wie sie z. B. in WO 98/46608, WO 99/41255 oder WO 03/004465 jeweils durch die allgemeine Formel I beschrieben werden,
- Amidfungizide wie Cyflufenamid sowie (Z)-N-[α-(Cyclopropylmethoxyimino)-2,3-difluoro-6-(difluoromethoxy)benzyl]-2-phenylacetamid.

Bevorzugte Mischungspartner des Pyraclostrobins sind: Metalaxyl, Dodemorph, Fenpropimorph, Fenpropidin, Guazatine, Spiroxamin, Tridemorph, Pyrimethanil, Cyrodinyl, Bitertanol, Bromoconazol, Cyproconazol, Difenoconazole, Dinitroconazol, Epoxiconazol, Fenbuconazol, Fluquinconazol, Flusilazol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol, Iprodion, Vinclozolin, Maneb, Mancozeb, Metiram, Thiram, Boscalid, Carbendazim, Carboxin, Oxycarboxin, Cyazofamid, Dithianon, Famoxadon, Fenamidon, Fenarimol, Flutolanil, Quinoxyfen, Thiophanat-methyl, Thiophanat-ethyl,Triforine, Dinocap Nitrophthal-isopropyl, Phenylpyrrole wie Fenpiclonil oder Fludioxonil, Acibenzolar-S-methyl, Benthiavalicarb, Carpropamid, Chlorothalonil, Cyflufenamid, Cymoxanil, Fenhexamid, Fentin-Acetat, Fenoxanil, Fluazinam, Fosetyl, Fosetyl-Aluminium, Iprovalicarb, Metrafenon, Zoxamid, Captan, Folpet, Dimethomorph, Azoxystrobin, Dimoxystrobin, Fluoxastrobin, Kresoxim-methyl, Metominostrobin, Orysastrobin, Picoxystrobin oder Trifloxystrobin.

Besonders bevorzugte Mischungspartner sind Metalaxyl, Fenpropimorph, Fenpropidin, Guazatine, Spiroxamin, Pyrimethanil, Cyrodinyl, Cyproconazol, Difenoconazole, Epoxiconazol, Fenbuconazol, Fluquinconazol, Flusilazol, Hexaconazol, Metconazol, Myclobutanil, Propiconazol, Prochloraz, Prothioconazol, Tebuconazol, Triticonazol, Iprodion, Vinclozolin, Boscalid, Carbendazim, Carboxin, Oxycarboxin, Cyazofamid, Dithianon, Quinoxyfen, Thiophanat-methyl, Thiophanat-ethyl,Dinocap, Nitrophthal-isopropyl, Fenpiclonil oder Fludioxonil, Benthiavalicarb, Carpropamid, Fenhexamid, Fenoxanil, Fluazinam, Iprovalicarb, Metrafenon, Zoxamid, Dimethomorph, Azoxystrobin, Dimoxystrobin, Fluoxastrobin, Kresoxim-methyl, Metominostrobin, Orysastrobin, Picoxystrobin oder Trifloxystrobin.

Ganz besonders bevorzugte Mischungspartner sind Fenpropimorph, Cyproconazol, Difenoconazole, Epoxiconazol, Fenbuconazol, Fluquinconazol, Flusilazol, Hexaconazol, Metconazol, Myclobutanil Propiconazol, Prochloraz, Prothioconazol, Tebuconazol, Triticonazol, Boscalid, Dithianon, Quinoxyfen, Thiophanat-methyl, Thiophanat-ethyl, Dinocap Fenpiclonil oder Fludioxonil, Benthiavalicarb, Carpropamid, Fenhexamid, Fenoxanil, Fluazinam, Iprovalicarb, Metrafenon, Zoxamid, Dimethomorph, Azoxystrobin, Dimoxystrobin, Fluoxastrobin, Kresoxim-methyl, Metominostrobin, Orysastrobin, Picoxystrobin oder Trifloxystrobin.

Durch die erfindungsgemäßen Formulierungen der kristallinen Modifikationen des Pyraclostrobins lassen sich grundsätzlich alle Schadpilzerkrankungen bekämpfen, die auch mit den bekannten Formulierungen des Pyraclostrobins bekämpft werden können. Abhängig von dem jeweiligen Mischungspartner handelt es sich beispielsweise um die folgenden Pflanzenerkrankungen:

- *Alternaria* Arten an Gemüse, Raps, Zuckerrüben, Soja, Getreide, Baumwolle, Obst und Reis (z.B. *A. solani* oder *A. alternata* an Kartoffel und anderen Pflanzen),
- *Aphanomyces* Arten an Zuckerrüben und Gemüse,
- *Ascochyta sp.* an Baumwolle und Reis,
- *Bipolaris*- und *Drechslera* Arten an Mais, Getreide, Reis und Rasen (z.B. D. *teres* an Gerste, *D. tritci-repentis* an Weizen),
- *Blumeria graminis* (Echter Mehltau) an Getreide,
- *Botrytis cinerea* (Grauschimmel) an Erdbeeren, Gemüse, Blumen und Weinreben,
- *Botryodiplodia sp.* an Baumwolle
- *Bremia lactucae* an Salat,
- *Cercospora* Arten an Mais, Sojabohnen, Reis und Zuckerrüben (z.B. C. *beticula* an Zuckerrüben),
- *Cochliobolus* Arten an Mais, Getreide, Reis (z.B. *Cochliobolus sativus* an Getreide, *Cochliobolus miyabeanus* an Reis),
- *Corynespora* sp. an Sojabohnen, Baumwolle und anderen Pflanzen
- *Colletotrichum* Arten an Sojabohnen, Baumwolle und anderen Pflanzen (z.B. C. *acutatum* an verschiedenen Pflanzen),
- *Curvularia sp.* an Getreide und Reis,
- *Diplodia sp.* an Getreide und Reis,
- *Exserohilum* Arten an Mais,
- *Erysiphe cichoracearumund Sphaerotheca fuliginea* an Gurkengewächsen,
- *Fusarium* und *Verticillium* Arten (z.B. *V. dahliae*) an verschiedenen Pflanzen (z.B. *F. graminearum* an Weizen),
- *Gaeumanomyces graminis* an Getreide,
- *Gibberella* Arten an Getreide und Reis (z.B. *Gibberella fujikuroi* an Reis),
- *Grainstaining complex* an Reis,
- *Helminthosporium* Arten (z.B. *H. graminicola*) an Mais und Reis,
- *Macrophomina sp.* an Soja und Baumwolle,
- *Michrodochium sp. z.B. M. nivale* an Getreide,
- *Mycosphaerella* Arten an Getreide, Bananen und Erdnüssen (*M. graminicola* an Weizen, M. *fijiesis* an Banane),
- *Phaeoisaripsis sp.* an Sojabohnen,
- *Phakopsara sp. z.b. P. pachyrhizi* und *Phakopsara meibomiae* an Sojabohnen,
- *Phoma sp.* an Soja,
- *Phomopsis* Arten an Sojabohnen, Sonnenblumen und Weinreben (*P. viticola* an Weinreben, *P. helianthii* an Sonnenblumen),

- *Phytophthora infestans* an Kartoffeln und Tomaten,
- *Plasmopara viticola* an Weinreben,
- *Penecilium sp.* an Soja und Baumwolle,
- *Podosphaera leucotricha* an Apfel,
- *Pseudocercosporella herpotrichoides* an Getreide,
- *Pseudoperonospora* Arten an Hopfen und Gurkengewächsen (z.B. *P. cubenis* an Gurke),
- *Puccinia* Arten an Getreide, Mais und Spargel (*P. triticina und P. striformis an* Weizen, *P. asparagi* an Spargel),
- *Pyrenophora* Arten an Getreide,
- *Pyricularia oryzae, Corticium sasakii, Sarocladium oryzae, S.attenuatum, Entyloma oryzae* an Reis,
- *Pyricularia grisea* an Rasen und Getreide,
- *Pythium spp.* an Rasen, Reis, Mais, Baumwolle, Raps, Sonnenblumen, Zuckerrüben, Gemüse und anderen Pflanzen,
- *Rhizoctonia-Arten* (z.B. *R. solani*) an Baumwolle, Reis, Kartoffeln, Rasen, Mais, Raps, Kartoffeln, Zuckerrüben, Gemüse und anderen Pflanzen,
- *Rynchosporium sp.* (*z.B. R. secalis*) an Reis und Getreide,
- *Sclerotinia* Arten (z.B. *S. sclerotiorum*) an Raps, Sonnenblumen und anderen Pflanzen,
- *Septoria tritici* und *Stagonospora nodorum* an Weizen,
- *Erysiphe* (syn. *Uncinulanecator*) an Weinrebe,
- *Setospaeria* Arten an Mais und Rasen,
- *Sphacelotheca reilinia* an Mais,
- *Thievaliopsis* Arten an Sojabohnen und Baumwolle,
- *Tilletia Arten* an Getreide,
- *Ustilago* Arten an Getreide, Mais und Zuckerrübe und
- *Venturia* Arten (Schorf) an Apfel und Birne (z.B. *V. inaequalis* an Apfel).

Die erfindungemäßen Modifikationen II und IV des Pyraclostrobins können in an sich bekannter Weise auch mit Insektizd-, Acaraizid oder Nematizid wirkenden Verbindungen formuliert werden. Insbesondere hat es sich als vorteilhaft erwiesen, Modifikationen II und IV des Pyraclostrobins zusammen mit wenigstens einem Wirkstoff einzusetzen, der gegen stechende, kauende, beißende oder saugende Insekten und sonstige Arthropoden z.B. aus der Ordnung der

- Coleoptera, insbesondere Phyllophaga sp. wie Phyllophaga cuyabana, Sternechus sp. wie Sternechus pingusi, Sternechuns subsignatus, Promecops sp. wie Promecops caricinollis, Aracanthus sp. wie Aracanthus morei, und Diabrotica sp. wie Diabrotica speciosa, Diabrotica Iongicornis, Diabrotica 12-punctata, Diabrotica virgifera, Oryzophagus sp.,
- Lepidoptera insbesondere Elasmopalpus sp. wie Elasmopalpus lignosellus, Di-Ioboderus sp.
- Isoptera, insbesondere Rhinotermitida,
- Homoptera, insbesondere Dalbulus maidis,

oder gegen Nematoden, einschließlich Wurzelknotennematoden, z.B. Meloidogyne spp. wie Meloidogyne hapla, Meloidogyne incognita, Meloidogyne javanica, und andere Meloidogyne species; Cysten bildende Nematoden wie Globodera rostochiensis und andere Globodera species; Heterodera avenae, Heterodera glycines, Heterodera schachtii, Heterodera trifolii, und andere Heterodera species; Gallennematoden z.B. Anguina species; Stengel- und Blattnematoden wie Aphelenchoides species, wirksam ist.

Beispielsweise kann man eine Formulierung, die eine erfindungsgemäße Modifikation II und/oder IV des Pyraclostrobins und Thiophanat-methyl oder ethyl enthält zur Bekämpfung der folgenden Schadpilze eingesetzt werden:

- *Alternaria sp.* Getreide, Baumwolle und Reis
- *Ascochyta sp. an* Baumwolle und Reis,
- *Botryodiplodia sp.* an Baumwolle,
- *Cercospora* Arten an Mais, Sojabohnen, Reis und anderen Pflanzen,
- *Corynespora* sp. an Sojabohnen, Baumwolle und anderen Pflanzen,
- *Colletotrichum* Arten an Sojabohnen, Baumwolle und anderen Pflanzen,
- *Curvularia sp.* an Getreide und Reis,
- *Diplodia sp.* an Getreide und Reis,
- *Drechslera sp.* an Getreide und Reis,
- *Fusarium sp.* an Getreide, Sojabohnen und Baumwolle,
- *Giberella sp.* an Getreide und Reis,
- *Macrophomia sp.* Sojabohnen und Baumwolle,

- *Penecilium sp.* an Sojabohnen und Baumwolle
- *Phaeoisaripsis sp.* an Sojabohnen,
- *Phoma sp.* an Sojabohnen,
- *Phomopsis sp.* an Sojabohnen,
- *Pythium sp.* an Sojabohnen und Baumwolle,
- *Pyrenophora sp.*
- *Pyricularia sp.* an Reis,
- *Rhizoctonia sp.* an Soja, Reis und Baumwolle,
- *Rhychosporium sp.* an Reis,
- *Septoria sp.* an Soja,
- *Tilletia sp.* an Getreide und Reis,
- *Ustilago sp.* an Getreide.

Beispielsweise kann man eine Formulierung, die eine erfindungsgemäße Modifikation II und/oder IV des Pyraclostrobins, Thiophanat-methyl oder ethyl und Fipronil oder einen anderen GABA-Antagonisten wie Acetoprole, Endosulfan, Ethiprole, Vaniliprole, Pyrafluprole oder Pyriprole enthält zur Bekämpfung der vorgenannten Schadpilze mit gleichzeitiger Bekämpfung von Insekten eingesetzt werden, z.B.

- Coleoptera, insbesondere Phyllophaga sp. wie Phyllophaga cuyabana, Sternechus sp. wie Sternechus pingusi, Sternechuns subsignatus, Promecops sp. wie Promecops carinicollis, Aracanthus sp. wie Aracanthus morei, und Diabrotica sp. wie Diabrotica speciosa, Diabrotica Iongicornis, Diabrotica 12-punctata, Diabrotica virgifera, Oryzophagus sp., und
- Lepidoptera insbesondere Elasmopalpus sp. wie Elasmopalpus lignosellus, Di-loboderus sp.

Beispielsweise kann man eine Formulierung, die eine erfindungsgemäße Modifikation II und/oder IV des Pyraclostrobins und Epoxyconazol enthält, zur Bekämpfung der folgenden Schadpilze eingesetzt werden:

- *Microdochium sp.* an Getreide.
- *Tilletia sp.* an Getreide und Reis,
- *Ustilago sp.* an Getreide.

Beispielsweise kann man eine Formulierung, die eine erfindungsgemäße Modifikation II und/oder IV des Pyraclostrobins, Triticonazol und Prochloraz oder Prochloraz-CuCl enthält, zur Bekämpfung der folgenden Schadpilze eingesetzt werden:

- *Microdochium sp.* an Getreide.
- *Tilletia sp.* an Getreide und Reis,
- *Ustilago sp.* an Getreide.

Die neuen Modifikationen II und IV des Pyraclostrobins erlauben die Herstellung lösungsmittelarmer bzw. lösungsmittelfreier wässriger Suspensionskonzentrate, sowohl des Pyraclostrobins alleine als auch von Pyraclostrobin mit anderen Pflanzenschutzwirkstoffe, insbesondere den zuvor angegebenen Mischungspartnern. Der Lösungsmittelgehalt, insbesondere der Gehalt an aromatischen Kohlenwasserstoffen beträgt, abzüglich etwaiger Frostschutzmittel, in der Regel nicht mehr als 2 Gew.-% des Suspensionskonzentrats und liegt häufig unterhalb 2 Gew.-%. Die erfindungsgemäßen Suspensionskonzentrate zeichnen sich insbesondere durch eine verbesserte Lagerstabilität im Vergleich zu den bekannten Suspensionskonzentraten und Suspoemulsionskonzentraten, die Pyraclostrobins enthalten.
Die folgenden Abbildungen und Beispiele dienen der Erläuterung der Erfindung und sind nicht einschränkend zu verstehen.

Abbildung 1: Röntgenpulverdiffraktogramm der Modifikation IV
Abbildung 2: Röntgenpulverdiffraktogramm der Modifikation II
Abbildung 3: Röntgenpulverdiffraktogramm der Modifikation I
Abbildung 4: Röntgenpulverdiffraktogramm der Modifikation III
Abbildung 5: Lichtmikroskopische Aufnahme der Formulierung aus Formulierbeispiel 1 nach 26 Wochen Lagerung bei 40°C.
Abbildung 6: Lichtmikroskopische Aufnahme der Formulierung aus Vergleichsformulierbeispiel nach 26 Wochen Lagerung bei 40°C.

Analytik:

Die Aufnahmen der Röntgenpulverdiffraktogramme wurden an einem D-5000 Diffraktometer der Fa. Siemens in Reflexionsgeometrie im Bereich von 2θ = 4° - 35° mit einer Schrittweite von 0,02° unter Verwendung von Cu-Kα-Strahlung bei 25°C durchgeführt. Aus ermittelten 2θ-Werten wurden die angegebenen Netzebenenabstände d berechnet.

Die Bestimmung der kristallographischen Daten der Modifikationen II und IV (Tabellen 1 und 2) erfolgte an einem Einkristalldiffraktometer der Fa. Siemens unter Verwendung von Cu-Kα-Strahlung.

Die Bestimmung der Schmelzpunkte und Schmelzwärmen erfolgte mittels DSC mit einem Simultaneous Thermal Analyzer STA 449 C Jupiter der Fa. NETZSCH mit einer Aufheizrate von 5 K/min im Bereich von -5° bis +80°C. Die Probenmenge betrug 5 bis 10 mg.

Die Bestimmung der Teilchengrößen in den Suspensionskonzentraten erfolgte mit einem Mastersizer 2000 der Fa. Malvern Instruments GmbH.

Herstellungsbeispiele

Beispiel 1: Herstellung von Pyraclostrobin Modifikation IV durch Kristallisation aus Isopropanol mit Impfkristallen der Modifikation IV

Ausführung

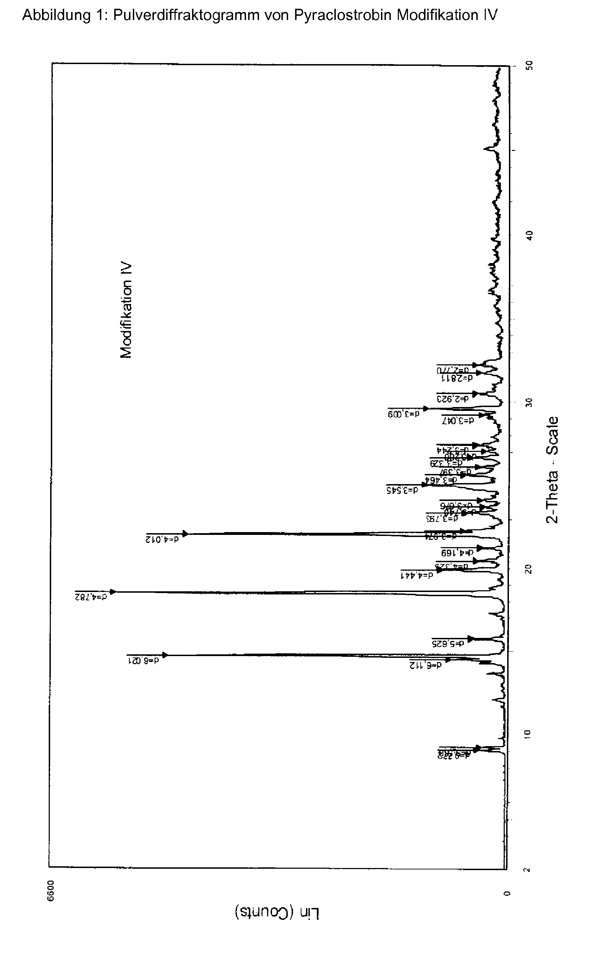

600 g Isopropanol werden auf 70°C erwärmt. 300 g amorphes Pyraclostrobin werden separat bei 80°C in eine dünnflüssige Schmelze übergeführt. Diese Schmelze wird unter kräftigem Rühren zu dem Isopropanol gegeben. Die Mischung wird bei 70°C gehalten, bis das Material vollständig gelöst vorliegt (ungefähr 30 Minuten). Anschließend lässt man die Mischung auf Zimmertemperatur abkühlen. Unter Rühren gibt man 1 g kristallines Pyraclostrobin der Modifikation IV zu. Nach etwa 1 Stunde setzt die Kristallisation ein. Die Mischung rührt man weitere 18 Stunden, trennt die Kristalle mittels Filtration ab und trocknet im Vakuum bei 25°C. Ausbeute: 290 g Pyraclostrobin. Die Modifikation IV wurde anhand ihrer Reflexe im Röntgenpulverdiffraktogramm identifiziert (Abbildung 1).

Beispiel 2: Herstellung von Pyraclostrobin Modifikation IV durch Kristallisation aus Ethanol mit Impfkristallen der Modifikation IV

1500 g Ethanol legt man in einem 2,5 l Gefäß mit Doppelwandung und Turbinenrührer (PBT-Turbine) vor und erwärmt auf 50°C. 1000 g Pyraclostrobin werden auf 70°C erwärmt und in das Reaktionsgefäß gegeben. Nach 10 min. Rühren bei 60°C lässt man die erhaltene klare Lösung langsam abkühlen. Bei 34°C gibt man 1 g Impfkristalle der Modifikation IV zu. Dann lässt man innerhalb von 114-116 Stunden auf Umgebungstemperatur abkühlen. Anschließend wird auf 10°C abgekühlt. Der Feststoff wird isoliert, mit 400 ml kaltem Ethanol gewaschen und im Vakuum (40 mbar) bei Umgebungstemperatur über einen Zeitraum von ca. 16 Stunden getrocknet. Ausbeute: 870 g (Schmelzpunkt 67°C). Die Modifikation IV wurde anhand ihrer Reflexe im Röntgenpulverdiffraktogramm identifiziert (siehe Abbildung 1).

Beispiel 3: Herstellung von Pyraclostrobin Modifikation IV durch Kristallisation aus Isopropanol ohne Impfkristalle

100 g Isopropanol werden auf 60°C erwärmt. Dann gibt man 15 g Pyraclostrobin zu und rührt solange, bis alles gelöst ist. Anschließend lässt man auf Umgebungstemperatur abkühlen und rührt noch 2 Wochen nach. Die Kristalle werden abfiltriert und im Vakuum bei Umgebungstemperatur 16 h getrocknet. Ausbeute: 12 g.

Das erhaltene Material zeigte in einer Röntgenpulverdiffraktometrie das in Abbildung 1 abgebildete Diagramm.

Beispiel 4: Herstellung von Pyraclostrobin Modifikation II durch Kristallisation aus Isopropanol ohne Impfkristalle

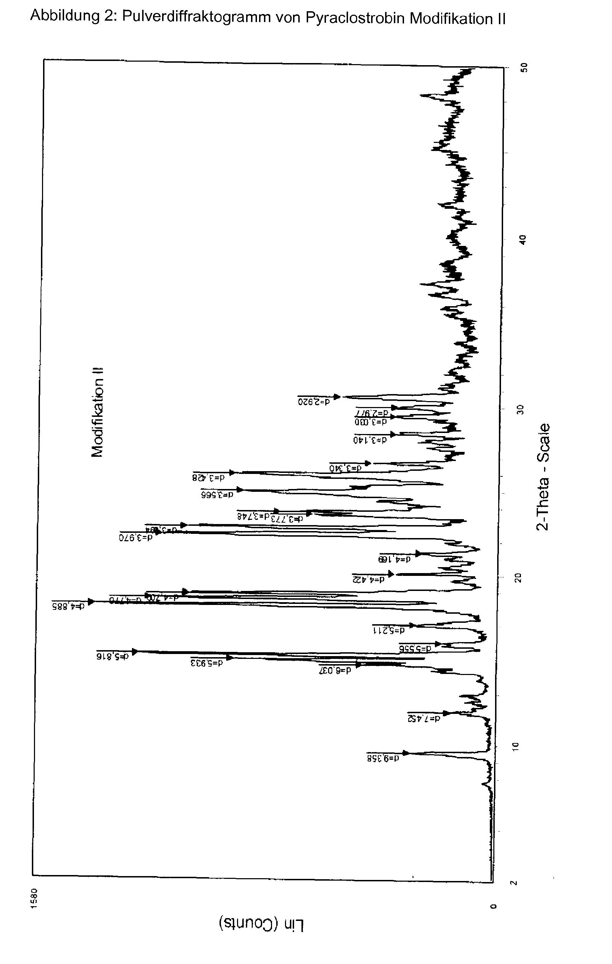

100 g Isopropanol werden auf 60°C erwärmt. Dann gibt man 15 g Pyraclostrobin unter Rühren zu und rührt bis alles gelöst ist. Anschließend lässt man auf 20°C abkühlen und rührt noch für 4 Stunden nach. Dann wird auf 10°C abgekühlt und eine Stunde weiter gerührt. Die Kristalle werden sofort isoliert und im Vakuum bei Umgebungstemperatur 16 h getrocknet. Ausbeute: 12 g. Das erhaltene Material zeigte in einer Röntgenpulverdiffraktometrie das in Abbildung 2 abgebildete Diagramm.

Beispiel 5: Herstellung von Pyraclostrobin Modifikation I durch Kristallisation aus der Schmelze

Amorphes Pyraclostrobin wurde geschmolzen und langsam abgekühlt. Die Modifikation I kristallisiert zuerst aus der Pyraclostrobinschmelze aus. Die Kristallisation wird durch Tempern bei ungefähr 40° bis 45°C beschleunigt.

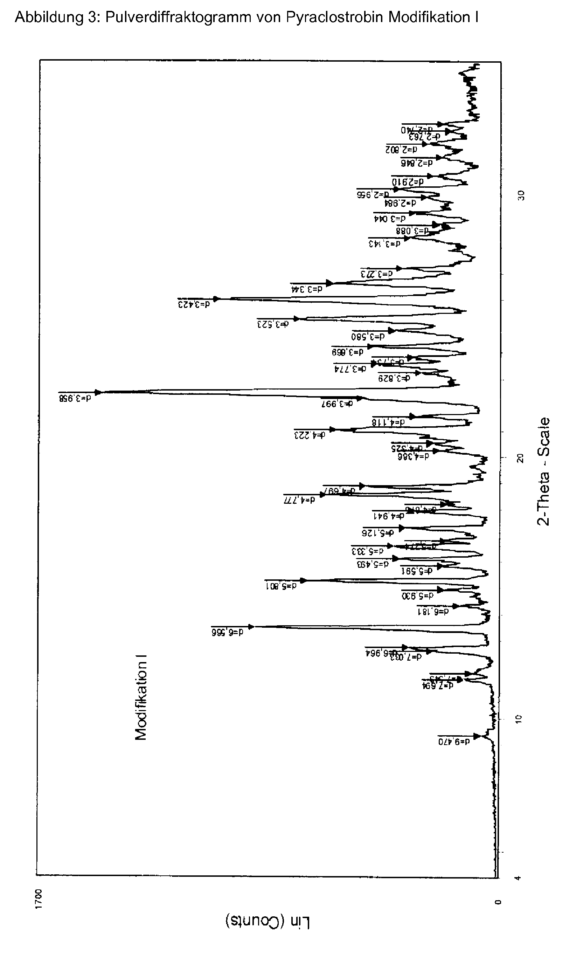

Das erhaltene Material weist das in Abbildung 3 abgebildete Röntgenpulverdiffraktogramm auf.

Beispiel 6: Herstellung von Pyraclostrobin Modifikation III durch Kristallisation aus der Schmelze

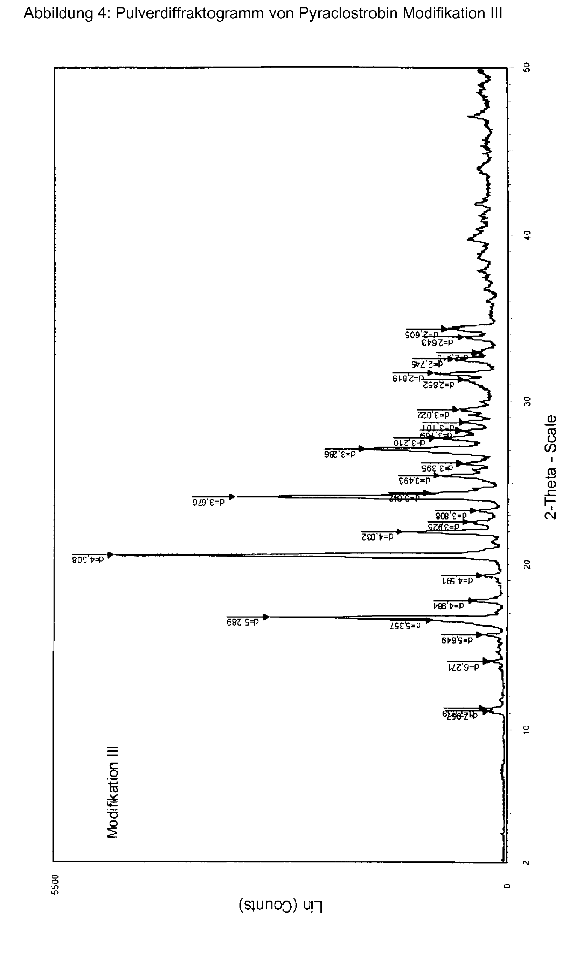

Diese Modifikation kristallisiert aus einer Pyraclostrobinschmelze nach einigen Wochen aus, wenn man die Schmelze bei einer Temperatur im Bereich von 18 bis 25°C belässt. Das erhaltene Material zeigte in einer Röntgenpulverdiffraktometrie das in Abbildung 4 abgebildete Diagramm.

Beispiel 7: Herstellung von Pyraclostrobin der Modifikation IV durch Kristallisation aus Ethanol in Gegenwart von Impfkristallen

358 g des Pyraclostrobin mit einer Reinheit von 99 % wurden bei 80°C verflüssigt und mit 525 g Ethanol (96%ig) verrührt, bis das Pyraclostrobin vollständig gelöst vorlag. Danach kühlte man innerhalb von 5 h auf 35°C und gab bei dieser Temperatur ca.1 g Impfkristalle der Modifikation IV hinzu. Dann kühlte man innerhalb von 3 h auf 20°C und fügte anschließend innerhalb 2 h 483 g Wasser hinzu. Nach Beendigung der Wasserzugabe rührt man noch eine Stunde bei 20°C und filtriert anschließend mittels einer Nutsche (Porosität 4). Nach dem Waschen mit 350 g Wasser wurde der erhaltene kristalline Feststoff im Vakuum bei 40°C getrocknet. Man erhielt 353 g eines kristallinen Produkts, das als Modifikation IV identifiziert wurde. Ausbeute: 98,6 %, Gehalt: 99,5 %, Schmelzpunkt: 63,0°C

Beispiel 8: Herstellung von Pyraclostrobin der Modifikation IV durch Kristallisation aus Methanol in Gegenwart von Impfkristallen

179,4 g des Wirkstoffes I wurden bei 80°C verflüssigt und mit 253 g Methanol (96%ig) verrührt, bis das Pyraclostrobin vollständig gelöst vorlag. Danach kühlte man innerhalb von 4 h auf 35°C und gab bei dieser Temperatur ca. 0,5 g Impfkristalle der Modifikation IV hinzu. Dann kühlte man innerhalb von 2 h auf 20°C und fügt anschließend innerhalb 1,5 h 252 g Wasser hinzu. Nach Beendigung der Wasserzugabe rührte man noch eine Stunde bei 20°C und filtrierte anschließend mittels einer Nutsche (Porosität 4). Nach dem Waschen mit 90 g Wasser wurde der erhaltene kristalline Feststoff im Vakuum bei 40°C getrocknet. Man erhielt 177,8 g eines kristallinen Produkts, das als Modifikation IV identifiziert wurde. Ausbeute: 99,1 %, Gehalt: 99,8 %, Schmelzpunkt: 65,0°C

Beispiel 9: Herstellung von Pyraclostrobin der Modifikation IV durch Kristallisation aus Methanol in Gegenwart von Impfkristallen

179,4 g des Wirkstoffes I wurden bei 80°C verflüssigt und mit 253 g Methanol (96%ig) verrührt, bis das Pyraclostrobin vollständig gelöst vorlag. Danach kühlte man innerhalb von 3 h auf 35°C und gab bei dieser Temperatur ca. 0,5 g Impfkristalle der Modifikation IV hinzu. Dann kühlte man innerhalb von 2 h auf 20°C und fügt anschließend innerhalb 1,5 h 252 g Wasser hinzu. Nach Beendigung der Wasserzugabe rührte man noch eine Stunde bei 20°C und filtrierte anschließend mittels einer Nutsche (Porosität 3). Nach dem Waschen mit 90 g Wasser wurde der erhaltene kristalline Feststoff abgetrennt. Man erhielt 236,4 g eines wasserfeuchten, kristallinen Produkts, das einen Wirkstoffgehalt von 75,3 % aufwies. Dies entspricht einer Ausbeute von 99,2 %. Eine Probe des erhaltenen Produkts wurde im Vakuum bei 40°C getrocknet. Auf diese Weise erhielt man ein kristallines Produkt, das als Modifikation IV identifiziert wurde und einen Schmelzpunkt von 65,2°C aufwies.

Vergleichsbeispiel 1: Kristallisation analog Beispiel 1, wobei man anstelle von Isopropanol Ethylacetat einsetzte, lieferte ein erstarrendes Öl und schlechte Ausbeuten an Pyraclostrobin.

Vergleichsbeispiel 2: Kristallisation analog Beispiel 1, wobei man im Unterschied zu der dort beschriebenen Methode keine Impfkristalle verwendete, lieferte ein erstarrendes Öl und schlechte Ausbeuten an Pyraclostrobin.

Formulierungsbeispiele

Vergleichsformulierbeispiel: Herstellung eines Suspensionskonzentrats von an Kieselsäure adsorbiertem Pyraclostrobin

Pyraclostrobin Premix

Wasser (ca. 60 Gew.-% der Gesamtformulierung) wird in einem geeigneten Gefäß vorgelegt. Dann werden das Netzmittel und anschließend die Kieselsäure eingerührt und die Mischung wird auf 80°C erwärmt. Anschließend gibt man 20 Gew.-Teile einer auf 80°C erwärmten Pyraclostrobinschmelze unter Rühren zu und rührt nach Beendigung der Zugabe noch weitere 30 Minuten bei 80°C. Danach wird die Suspension unter Rühren auf Umgebungstemperatur abgekühlt. Der Premix wies die folgende Zusammensetzung auf:

| Wasser | 60 Gew.-Teile | |
|---|---|---|
| Netzmittel | 5 Gew.-Teile | (Naphthalinsulfonsäure-Formaldehyd-Kondensationsprodukt) |
| Kieselsäure | 15 Gew.-Teile | (Fällungskieselsäure) |
| Pyraclostrobin | 20 Gew.-Teile | |

Endformulierung:

Wasser wird in einem geeigneten Gefäß vorgelegt. Dann gibt man unter Rühren Netzmittel, Dispergiermittel, Frostschutzmittel, Stabilisator und Teilmengen des Entschäumers zu. Hierzu gibt man den zweiten Wirkstoff und das Pyraclostrobin Premix. Anschließend wird die Dispersion in einer Perlmühle unter effektivem Kühlen auf die gewünschte Feinheit vermahlen. Dann wird die Formulierung unter Zugabe der restlichen Formulierhilfsstoffe (Bakterizid, Andicker, restlicher Entschäumer) fertig gestellt. Die Endformulierung wies die folgende Zusammensetzung auf:

| Wasser | 42,3 Gew.-Teile | |
|---|---|---|
| Netzmittel | 4 Gew.-Teile | (Naphthalinsulfonsäure-Formaldehyd-Kondensationsprodukt) |
| Kieselsäure | 7,5 Gew.-Teile | (Fällungskieselsäure) |
| Pyraclostrobin | 10 Gew.-Teile | |
| Dispergiermittel | 3 Gew.-Teile | (EO-PO-Blockcopolymer) |
| Frostschutzmittel | 2 Gew.-Teile | (Propylenglykol) |
| Entschäumer | 0,5 Gew.-Teile | (handelsüblicher Silikonentschäumer, z.B. Silfoam Typen der Firma Wacker) |
| Stabilisator | 0,2 Gew.-Teile | (Puffersystem) |
| Wirkstoff | 30 Gew.-Teile | (Folpet) |
| Bakterizid | 0,2 Gew.-Teile | (substituierte Isothiazolin-3-one) |
| Andicker | 0,3 Gew.-Teile | (xanthan gum) |

Formulierbeispiel 1

Herstellung eines Suspensionskonzentrates aus Pyraclostrobin in der Modifikation IV

Die Restwassermenge wird in einem geeigneten Gefäß vorgelegt. Dann werden die weiteren Formulierungsbestandteile: Netzmittel, Dispergiermittel, Frostschutzmittel, Stabilisator und Teilmengen des Entschäumers eingerührt. Dazu gibt man kristallines Pyraclostrobin und den zweiten festen Wirkstoff. Anschließend wird die Dispersion in einer Perlmühle unter effektivem Kühlen auf die gewünschte Feinheit vermahlen. Dann wird die Formulierung unter Zugabe der restlichen Formulierhilfsstoffe fertig gestellt.

| Wasser | 46,9 Gew.-Teile | |
|---|---|---|
| Netzmittel | 3 Gew.-Teile | (Naphthalinsulfonsäure-Formaldehyd-Kondensationsprodukt) |
| Pyraclostrobin | 10 Gew.-Teile | |
| Dispergiermittel | 2 Gew.-Teile | (Polyethylenoxid-Polypropylenoxid-Blockcopolymer (EO-PO-Blockcopolymer)) |
| Frostschutzmittel | 7 Gew.-Teile | (Propylenglykol) |
| Entschäumer | 0,5 Gew.-Teile | (handelsüblicher Silikonentschäumer, z.B. Silfoam Typen der Firma Wacker) |
| Stabilisator | 0,1 Gew.-Teile | (Puffersystem) |
| Wirkstoff | 30 Gew.-Teile | (Folpet) |
| Bakterizid | 0,2 Gew.-Teile | (substituierte Isothiazolin-3-one) |
| Andicker | 0,3 Gew.-Teile | (xanthan gum) |

Zur Bestimmung der Stabilität wurden die Formulierungen über den in Tabelle 3 angegebenen Zeitraum bei 40°C gelagert. Zur Bestimmung der Teilchengröße mittels Lichtstreuung wurde eine Probe in Wasser verdünnt und dispergiert und dann mit dem Mastersizer 2000 die Teilchengrößenverteilung bestimmt.

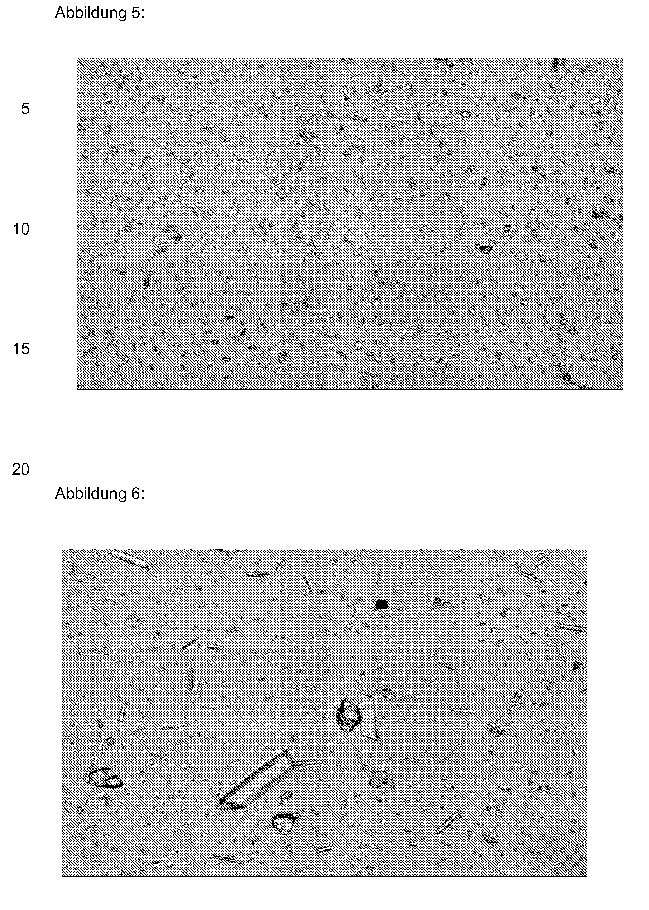

Die in den Abbildungen 5 und 6 gezeigten lichtmikroskopischen Aufnahmen einer 5%igen Verdünnung wurden an einem Leica Mikroskop mit einem 3 CCD Color Vision Camera Modul gemacht.

Zur Bestimmung der Dispersionsstabilität wurde eine 2%ige Verdünnung in einem 100 ml Spitzzylinder hergestellt. Das Volumen des sich bildenden Sediments wurde nach einer Standzeit von 2 Stunden abgelesen.

Tabelle 3: Lagerstabilität

|  | Vergleichsbeispiel | Formulierbeispiel 1 |
|---|---|---|
| **Dispersionsstabilität 2 %, 2h** |  |  |
| nach 4 Wochen Lagerung, 40°C | 0,4 ml Bodensatz | 0,15 ml Bodensatz |
| nach 26 Wochen Lagerung, 40°C | 0,5 ml Bodensatz | 0,3 ml Bodensatz |
| **Korngrößenverteilung** |  |  |
| nach 4 Wochen Lagerung, 40°C | | |
| < 2 $\mu$m [1] | 56% | 65% |
| 100 % < [2] | 95 $\mu$m | 24 $\mu$m |
| nach 26 Wochen Lagerung, 40°C | | |
| < 2 $\mu$m | 42 % | 57 % |
| 100 % < | 172 $\mu$m | 21 $\mu$m |
| 1) Gew.-% Teilchen unterhalb 2 $\mu$m 2) Maximale Teilchengröße | | |

In zu Beispiel 1 analoger Weise wurden die folgenden wässrigen Suspensionskonzentrate hergestellt:

Beispiel 3:

| | | |
|---|---|---|
| Wasser | 42 Gew.-Teile | |
| Netzmittel | 2,6 Gew.-Teile | (Naphthalinsulfonsäure-Formaldehyd-Kondensationsprodukt) |
| Pyraclostrobin | 4 Gew.-Teile | |
| Dispergiermittel | 2,7 Gew.-Teile | (EO-PO-Blockcopolymer) |
| Frostschutzmittel | 6,3 Gew.-Teile | (Propylenglykol) |
| Entschäumer | 0,5 Gew.-Teile | (handelsüblicher Silikonentschäumer, z.B. Silfoam Typen der Firma Wacker) |
| Stabilisator | 1,4 Gew.-Teile | (Puffersystem) |
| Wirkstoff | 40 Gew.-Teile | (Folpet) |
| Bakterizid | 0,2 Gew.-Teile | (substituierte Isothiazolin-3-one) |
| Andicker | 0,3 Gew.-Teile | (xanthan gum) |

Beispiel 4:

| | | |
|---|---|---|
| Wasser | 47 Gew.-Teile | |
| Netzmittel | 2 Gew.-Teile | (Naphthalinsulfonsäure-Formaldehyd-Kondensationsprodukt) |

(fortgesetzt)

| Pyraclostrobin | 40 Gew.-Teile | |
| Dispergiermittel | 3 Gew.-Teile | (EO-PO-Blockcopolymer) |
| Frostschutzmittel | 7 Gew.-Teile | (Propylenglykol) |
| Entschäumer | 0,5 Gew.-Teile | (handelsüblicher Silikonentschäumer, z.B. Silfoam Typen der Firma Wacker) |
| Bakterizid | 0,2 Gew.-Teile | (substituierte Isothiazolin-3-one) |
| Andicker | 0,3 Gew.-Teile | (xanthan gum) |

Beispiel 5

| Wasser | 47,1 Gew.-Teile | |
| Netzmittel | 2 Gew.-Teile | (Naphthalinsulfonsäure-Formaldehyd-Kondensationsprodukt) |
| Pyraclostrobin | 10 Gew.-Teile | |
| Dispergiermittel | 3 Gew.-Teile | (EO-PO-Blockcopolymer) |
| Frostschutzmittel | 7 Gew.-Teile | (Propylenglykol) |
| Entschäumer | 0,5 Gew.-Teile | (handelsüblicher Silikonentschäumer, z.B. Silfoam Typen der Firma Wacker) |
| Wirkstoff | 20 Gew.-Teile | (Boscalid) |
| Bakterizid | 0,2 Gew.-Teile | (substituierte Isothiazolin-3-one) |
| Andicker | 0,2 Gew.-Teile | (Xanthan gum) |

Die Ergebnisse der Stabilitätsuntersuchungen sind in Tabelle 4 zusammengestellt.

Tabelle 4: Lagerstabilität

| | Beispiel 3 | Beispiel 5 |
|---|---|---|
| **Dispersionsstabilität 2 %, 1 h** direkt nach Herstellung | Spur Bodensatz | Spur Bodensatz |
| nach 4 Wochen Lagerung, 20°C | Spur Bodensatz | Spur Bodensatz |
| 40°C | 0,05 ml Bodensatz | Spur Bodensatz |
| nach 26 Wochen Lagerung, 20°C | Spur Bodensatz | Spur Bodensatz |
| 40° C | 0,05 ml Bodensatz | Spur Bodensatz |
| **Korngrößenverteilung** | | |
| direkt nach Herstellung | | |
| < 2 $\mu$m [1]/100 % < [2] nach 4 Wochen Lagerung bei 20°C | 76 %/11 $\mu$m | 72 %/11 $\mu$m |
| < 2 $\mu$m [1]/100 % < [2] bei 40°C | 68 %/21 $\mu$m | 63 %/15 $\mu$m |
| < 2 $\mu$m [1]/100 % < [2] | 66 %/21 $\mu$m | 40 %/21 $\mu$m |
| nach 26 Wochen Lagerung, bei 20°C < 2 $\mu$m [1]/100 % < [2] bei 40°C | 67 %/24 $\mu$m | 56 %/21 $\mu$m |
| < 2 $\mu$m [1]/100 % < [2] | 44 %/33 $\mu$m | 33 %/28 $\mu$m |
| 1) Gew.-% Teilchen unterhalb 2 $\mu$m 2) Maximale Teilchengröße | | |

**Patentansprüche**

1. Kristalline Modifikation II des Pyraclostrobins, die in einem Röntgenpulverdiffraktogramm bei 25°C wenigstens vier der folgenden Reflexe zeigt:

$$d = 5,93 \pm 0,01 \text{ Å}$$
$$d = 5,82 \pm 0,01 \text{ Å}$$
$$d = 4,89 \pm 0,01 \text{ Å}$$
$$d = 4,78 \pm 0,01 \text{ Å}$$
$$d = 4,71 \pm 0,01 \text{ Å}.$$
$$d = 3,97 \pm 0,01 \text{ Å}.$$
$$d = 3,89 \pm 0,01 \text{ Å}.$$
$$d = 3,77 \pm 0,01 \text{ Å}.$$
$$d = 3,75 \pm 0,01 \text{ Å}.$$
$$d = 3,57 \pm 0,01 \text{ Å}.$$
$$d = 3,43 \pm 0,01 \text{ Å}.$$

2. Kristalline Modifikation nach Anspruch 15 mit einem Schmelzpunkt im Bereich von 57 bis 58°C.

3. Kristalline Modifikation nach Anspruch 1 oder 2 mit einem Gehalt an Pyraclostrobin von wenigstens 98 Gew.-%.

4. Verfahren zur Herstellung einer kristallinen Modifikation II des Pyraclostrobins gemäß einem der Ansprüche 1 bis 3, umfassend die folgenden Schritte:

   i) Lösen von amorphem Pyraclostrobin in einem organischen Lösungsmittel, das wenigstens 50 Vol.-% wenigstens eines $C_1$-$C_4$-Alkanols enthält; und
   ii) Bewirken einer Kristallisation von Pyraclostrobin über einen Zeitraum von weniger als 10 h in Abwesenheit von Impfkristallen der Modifikation IV.

5. Mittel für den Pflanzenschutz, enthaltend Pyraclostrobin in Form der Modifikation II nach einem der Ansprüche 1 bis 3 sowie übliche Trägerstoffe und/oder Hilfsmittel.

6. Mittel nach Anspruch % in Form eines wässrigen Suspensionskonzentrats.

7. Verwendung von Pyraclostrobin in Form der Modifikation II nach einem der Ansprüche 1 bis 3 zur Bekämpfung von pflanzenpathogenen Pilzen.

Abbildung 1: Pulverdiffraktogramm von Pyraclostrobin Modifikation IV

Abbildung 2: Pulverdiffraktogramm von Pyraclostrobin Modifikation II

Abbildung 3: Pulverdiffraktogramm von Pyraclostrobin Modifikation I

Abbildung 4: Pulverdiffraktogramm von Pyraclostrobin Modifikation III

Abbildung 5:

Abbildung 6:

Europäisches
Patentamt

European
Patent Office

Office européen
des brevets

# EUROPÄISCHER RECHERCHENBERICHT

**Nummer der Anmeldung**

EP 10 17 5103

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 03/082013 A (BASF AG [DE]) 9. Oktober 2003 (2003-10-09) * Ansprüche 1,4; Tabellen 1,2 * ----- | 1-7 | INV. A01N47/24 C07D231/22 |
| | | | **RECHERCHIERTE SACHGEBIETE (IPC)** |
| | | | A01N C07D |

Der vorliegende Recherchenbericht wurde für alle Patentansprüche erstellt

| Recherchenort | Abschlußdatum der Recherche | Prüfer |
|---|---|---|
| München | 28. Januar 2011 | Seelmann, Ingo |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 10 17 5103

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-01-2011

| Im Recherchenbericht angeführtes Patentdokument | Datum der Veröffentlichung | Mitglied(er) der Patentfamilie | Datum der Veröffentlichung |
|---|---|---|---|
| WO 03082013 A | 09-10-2003 | AT 375725 T | 15-11-2007 |
| | | AU 2003221518 A1 | 13-10-2003 |
| | | BR 0308733 A | 04-01-2005 |
| | | CA 2480241 A1 | 09-10-2003 |
| | | CN 1642425 A | 20-07-2005 |
| | | CO 5611069 A2 | 28-02-2006 |
| | | DE 60316926 T2 | 31-07-2008 |
| | | EP 1492406 A1 | 05-01-2005 |
| | | JP 2005521703 T | 21-07-2005 |
| | | MX PA04008651 A | 08-09-2005 |
| | | NZ 535337 A | 31-03-2006 |
| | | ZA 200408697 A | 10-11-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9601256 A
- WO 03082013 A
- WO 9846608 A
- WO 9941255 A
- WO 03004465 A

### In der Beschreibung aufgeführte Nicht-Patentliteratur

- **Herms, S. ; Seehaus, K. ; Koehle, H. ; Conrath, U.** Pyraclostrobin - "More than just a Fungicide". *Phytomedizin,* 2002, vol. 32, 17
- Handbook of Chemistry and Physics. CRC Pres
- Ullmanns Encyclopedia of Industrial Chemistry. Wiley-VCH, 1997
- Industrial Solvents Handbook. Marcel Dekker, 2003
- **M. Zlokarnik, Stirring.** Ullmann's Encyclopedia of Industrial Chemistry. Wiley-VCH, 1997